# EUROPEAN PATENT APPLICATION

(11) **EP 4 800 111 A1**
(43) Date of publication of application: **02.09.2026**
(21) Application number: 23956345.5
(22) Date of filing: 24.10.2023
(51) Int. Cl.: C12N 15/47, C12N 15/62, C12N 15/85, C12N 5/10, A61K 39/205, A61P 31/14

(54) **MRNA VACCINE AGAINST RABIES VIRUSES AND PREPARATION METHOD THEREFOR**

(71) Applicant: CSPC Megalith Biopharmaceutical Co., Ltd., Shijiazhuang, Hebei 050025 (CN)
(72) Inventor: MA, Chengxin, Shijiazhuang, Hebei 050025 (CN); WANG, Lingyu, Shijiazhuang, Hebei 050025 (CN); FAN, Chao, Shijiazhuang, Hebei 050025 (CN); WEI, Lifan, Shijiazhuang, Hebei 050025 (CN); YANG, Sicong, Shijiazhuang, Hebei 050025 (CN); WU, Leibin, Shijiazhuang, Hebei 050025 (CN); HU, Xuewen, Shijiazhuang, Hebei 050025 (CN); DAN, Mo, Shijiazhuang, Hebei 050025 (CN); LU, Yanli, Shijiazhuang, Hebei 050025 (CN); LYU, Lu, Shijiazhuang, Hebei 050025 (CN); WANG, Yajuan, Shijiazhuang, Hebei 050025 (CN); LI, Yanhui, Shijiazhuang, Hebei 050025 (CN); SUN, Zhaopeng, Shijiazhuang, Hebei 050025 (CN)
(74) Representative: Gille Hrabal Partnerschaftsgesellschaft mbB Patentanwälte
(86) International application number: PCT/CN2023/126187
(87) International publication number: WO 2025/086091

(57) **Abstract**

The present application belongs to the technical field of biological medicines, and particularly relates to a mRNA vaccine against rabies viruses and a preparation method therefor, the nucleotide sequence of said vaccine being shown as any one of SEQ ID NO. 1-3. The mRNA vaccine obtained by the present application can generate neutralizing antibodies at a high level, has broad-spectrum neutralizing activity on representative strains of seven pandemic populations of rabies viruses in China, effectively induces B cell immunity and T cell immunity, and provides good protection effects for mice challenged with viruses in pre-exposure and post-exposure immune tests. In addition, the vaccine can be preserved stably for a long period under a condition of -20 °C, thereby satisfying the requirements of use in countries and regions of various economic conditions.

## Description

### TECHNICAL FIELD

The present application relates to the technical field of biomedicine, and in particular to a rabies virus mRNA vaccine and a preparation method therefor.

### BACKGROUND

Rabies virus (RABV) belongs to the *Lyssavirus* genus of the *Rhabdoviridae* family, and is a single-stranded RNA virus. Rabies virus is bullet-shaped, has a helical nucleocapsid, has an envelope on the surface, and contains a single-stranded negative-strand RNA. The genome size of rabies virus is about 12 Kb, and the genome mainly encodes 5 proteins, i.e., glycoprotein (G protein), nucleocapsid protein, phosphoprotein, matrix protein, and transcriptase protein. Rabies virus relies on the G protein on its membrane surface to bind to the acetylcholine receptor on the surface of nerve cells and invade the cells, thereby causing serious damage to the central nervous system of humans or animals and eventually leading to the death of the host. Studies have shown that rabies virus can infect a variety of mammals, about 95% of human infections are caused by dog bites, and other hosts include coyotes, skunks, bats, and the like. Based on the latest data, 60,000 people die from rabies each year, about 1 person every 10 min.

The complete rabies virus genome can express various proteins such as glycoprotein (G protein), nucleocapsidprotein (N protein), phosphoprotein (P protein), matrix protein (M protein), and transcriptase protein. However, only the G protein is considered to be a protein capable of inducing the body to produce neutralizing antibodies, and the immune effect caused by the G protein is equivalent to that of the whole virus. Moreover, the G protein has an important function in the physiological cycle of rabies virus, that is, the G protein can form a homotrimeric spike structure on the rabies virus capsid, and this hollow structure can allow signaling molecules and genetic material to pass through, which is an important condition for virus uncoating. Thus, the G protein is generally selected as a preferred antigen protein for vaccines.

Specifically, the G protein has a full length of 1575 bp and comprises an open reading frame (ORF) of 524 amino acid residues, wherein the mature G protein comprises 505 amino acid residues, and the remaining 19 amino acid residues constitute a G protein signal peptide, which is cleaved by a protease during the maturation of the G protein. The G protein is present on the surface of the virus particle as a trimeric spike. Moreover, there are about 1600-1900 spikes with a size of 10 nm on the surface of each particle. The G protein can be structurally divided into an extracellular region (amino acid residues at positions 1-439), a transmembrane region (amino acid residues at positions 440-461), and an intracellular region (amino acid residues at positions 462-505), which play important roles in receptor recognition, membrane fusion, the stability of G protein spatial conformation, and the like. The extracellular region contains three important antigenic determinant sites, i.e., the GI region, the G II region, and the G III region, wherein the G I region contains a spatial conformational antigenic epitope and a linear antigenic epitope; the G II region contains two discontinuous spatial conformational epitopes; and the G III region contains one spatial conformational epitope and contains recognition sites for B cells and T cells. The G protein is capable of inducing host cells to generate specific immune responses, and is also capable of stimulating the proliferation of cytotoxic T lymphocytes (CTLs) and helper T cells.

Due to the extremely strong affinity of rabies virus for nervous tissues, the mortality rate is almost 100% in the case of infection, and no effective treatment is available at present. In addition, China has the second highest incidence of rabies after India. Moreover, due to the continuous increase in the number of breeding dogs and the lack of awareness of epidemic prevention, rabies always seriously threatens the life and health of people in China. In conclusion, vaccination is still the most effective method for preventing rabies at present, and the development of a lower-cost and more effective rabies vaccine is still of great social and economic value.

At present, the types of human rabies vaccines approved for marketing in China include Vero cell purified vaccines, human diploid cell vaccines, and hamster kidney primary cell purified vaccines. The main advantages of the vaccines described above are that the technology is relatively mature, the incidence of adverse effects is low, and the immune effect is good. However, the disadvantages of the vaccines are also significant, mainly including the following:
1. Attenuated vaccines have safety concerns, and the risk of infection exists if the attenuation is not complete.
2. Traditional vaccine preparation processes are complicated and require contact with live viruses in the production process, which has certain potential safety hazards.
3. The traditional vaccine production involves a cell culture stage, which requires high levels of quality control and process scale-up technology, posing a risk of causing incidents in product quality.
4. Traditional vaccines produce low amounts of effective antigens, and in order to ensure the effect, the virus titer often needs to be increased, thereby increasing the production cost.

In contrast, mRNA vaccines have unique advantages:
1. mRNA vaccines are a non-infectious and non-integrating platform that does not directly inject complete viral genetic materials or viral particles into the body, and do not have the potential risk of confirmed infection or insertion mutation. mRNA vaccines can be quickly metabolized and degraded by normal cells, and therefore, the safety is guaranteed;
2. mRNA vaccines are capable of effectively activating T cell immunity and B cell immunity, and have better effectiveness than that of inactivated vaccines;
3. The product design and production process of mRNA vaccines are relatively simple, and the large-scale production difficulty and production cost are relatively low.

At present, for rabies mRNA vaccines, some products have entered the clinical research stage. For example, CureVac AG company developed an mRNA vaccine CV7201 against rabies virus, but the clinical trials of this product have been suspended at present. Another product CV7202 (NCT03713086) from CureVac AG company is also not progressing smoothly. As can be seen, there are few mRNA vaccine products for rabies virus vaccines at present.

In conclusion, the immunization procedures of the currently available commercial rabies vaccines are cumbersome, the number of injections is large, and the patient compliance is poor. Therefore, there is an unmet medical need to develop a safe and effective rabies vaccine that is more affordable and prepared faster than currently available vaccines. In addition, mRNA vaccine products against rabies virus in the prior art are also extremely scarce, and more effective mRNA vaccine products with lower side effects are urgently needed.

In order to overcome the defects in the prior art, after years of research, the inventors of the present patent select protein G encoded by rabies virus as an antigen, and by optimizing and designing an mRNA sequence, develop an mRNA vaccine that can effectively prevent rabies virus infection in animals, can simultaneously induce the humoral immunity and cellular immunity, and has neutralizing antibodies at a high concentration in the body for up to 1 year, thereby providing a long-acting immune protection effect against rabies virus and achieving unexpected technical effects.

### SUMMARY

**In a first aspect**, the present application provides an immune composition (e.g., an mRNA vaccine) comprising an RNA encoding a highly immunogenic antigen capable of eliciting an effective neutralizing antibody response against a rabies virus antigen (e.g., glycoprotein G).

In some embodiments, the mRNA comprises a coding region encoding at least one antigen peptide or protein derived from glycoprotein G (RAV-G or RABV-G), nucleocapsidprotein N (RAV-N), phosphoprotein P (RAV-P), matrix protein M (RAV-M), or RNA polymerase L (RAV-L) of rabies virus or a fragment or variant thereof.

In some embodiments, the mRNA comprises a coding region encoding a full length or a portion of SEQ ID NO. 4 or an amino acid sequence having 50%, 60%, 70%, 80%, 85%, 86%, 87%, 88%, 89%, 90%, 91%, 92%, 93%, 94%, 95%, 96%, 97%, 98%, or 99% identity to SEQ ID NO. 4.

**In a second aspect**, the present application provides a polynucleotide (e.g., an mRNA) encoding at least one antigen peptide or protein derived from rabies virus or an immunogenic fragment or an immunogenic variant thereof, wherein the polynucleotide comprises at least one heterologous untranslated region (UTR), which comprises a nucleotide sequence having at least 80%, at least 85%, at least 90%, at least 95%, at least 98%, or 100% identity to the nucleotide sequence of any one of SEQ ID NOs: 1-3.

In some embodiments, the polynucleotide is suitable for use in producing a vaccine.

In some embodiments, the at least one antigen peptide or protein comprises or consists of at least one fragment or immunogenic variant derived from a structural protein, a helper protein, or a replication protein, or any of the proteins described above.

In some embodiments, the antigen peptide or protein is selected from at least one antigen peptide or protein of glycoprotein G (RAV-G or RABV-G), nucleocapsidprotein N (RAV-N), phosphoprotein P (RAV-P), matrix protein M (RAV-M), or RNA polymerase L (RAV-L) of rabies virus or a fragment or variant thereof.

In some embodiments, the polynucleotide of the present application may comprise or consist of a nucleotide sequence having at least 75%, at least 80%, at least 90%, at least 95%, at least 96%, at least 97%, at least 98%, at least 99%, or 100% identity to any one of SEQ ID NOs.: 1, 2, and 3.

In some embodiments, at least one coding region of the polynucleotide of the present application comprises one or more mutations, and the coding region comprises a nucleotide sequence optimized for G/C content.

In some embodiments, the heterologous untranslated region (UTR) comprises at least one heterologous 3' UTR and/or 5' UTR.

In some embodiments, the polynucleotide comprises at least the following structures:
(a) a 5'-cap structure;
(b) a 3' polyadenylic acid sequence, preferably 50-200 As, and more preferably 80-200 As;
(c) a 5' UTR having a length of 10-200 nucleotides, preferably 15-150 nucleotides, preferably alpha-1-globin 5' UTR or KOZAK sequences;
(d) a 3' UTR, preferably including 3' UTR sequences of human gp130, DH143, hHBB, and hHBA1;
(e) an optimized mRNA sequence having a higher GC base content than that of a natural sequence (wild type).

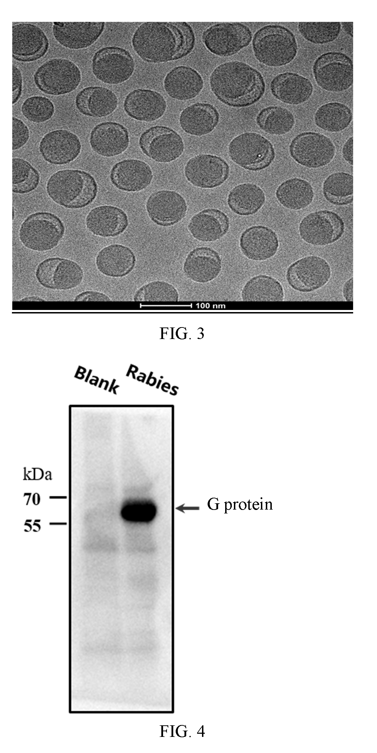

In some embodiments, the 3' UTR may also be selected from 3' UTRs of PSMB3, ALB7, α-globin, CASP1, COX6B1, GNAS, NDUFA1, DH143, gp130, hHBB, hHBA1, CYBA (cytochrome b-245 alpha chain), rabbit β-globin, hepatitis B virus (HBV), VEEV (Venezuelan equine encephalitis virus) virus, rps9 (ribosomal protein S9), FIG4 (FIG4 phosphoinositide 5-phosphatase), human albumin hHBB (human hemoglobin subunit beta), and HBA1 (human hemoglobin subunit alpha 1), or a homolog, fragment, or variant from any one of these genes.

In some embodiments, the 5' UTR is selected from 5' UTRs of *Xenopus laevis* or human α-globin or β-globin, human cytochrome b-245a polypeptide, hydroxysteroid (17b) dehydrogenase, tobacco etch virus, α-1-globin, HSD17B4, RPL32, ASAH1, ATP5A1, MP68, NDUFA4, NOSIP, RPL31, SLC7A3, TUBB4B, and UBQLN2, or a homolog, fragment, or variant from any one of these genes.

In some embodiments, the KOZAK sequence is set forth in SEQ ID NO. 5.

In some embodiments, the alpha-1-globin 5'-UTR sequence is the nucleotide sequence set forth in SEQ ID NO. 6.

In some embodiments, the 3' UTR sequence comprises the nucleotide sequence set forth in SEQ ID NO: 8 and/or 9.

In some embodiments, the 3'-polyadenylic acid sequence is preferably 60-120 As, more preferably 80-110 As, and further preferably 100 As, as set forth in SEQ ID NO. 7.

In some embodiments, the mRNA further comprises a 5' guanosine cap selected from: m7Gppp(2'OMeA)pG, m7GpppApA, m7GpppApC, m7GpppApG, m7GpppApU, m7GpppCpA, m7GpppCpC, m7GpppCpG, m7GpppCpU, m7GpppGpA, m7GpppGpC, m7GpppGpG, m7GpppGpU, m7GpppUpA, m7GpppUpC, m7GpppUpG, m7GpppUpU, m7Gpppm6ApG, m7G3'OmepppApA, m7G3'OmepppApC, m7G3'OmepppApU, m7G3'OmepppApG, m7G3'OmepppCpA, m7G3'OmepppCpC, m7G3'OmepppCpG, m7G3'OmepppCpU, m7G3'OmepppUpA, m7G3'OmepppUpC, m7G3'OmepppUpG, m7G3'OmepppUpU, m7G3'OmePppA2'OmepG, m7G3'OmepppA2'OmepC, m7G3'OmepppA2'OmepU, m7G3'OmepppA2'OmepA, m7G3'OmepppC2'OmepA, m7G3'OmepppC2'OmepU, m7G3'OmepppC2'OmepG, m7G3'OmepppC2'OmepC, m7G3'OmepppG2'OmepA, m7G3'OmepppG2'OmepU, m7G3'OmepppG2'OmepG, m7G3'OmepppG2'OmepC, m7G3'OmepppU2'OmepA, m7G3'OmepppU2'OmepU, m7G3'OmepppU2'OmepG, and m7G3'OmepppU2'OmepC. In some embodiments, the mRNA comprises a chemically modified base or analog thereof, including 5-methoxymethyl uridine, 5-methylthio uridine, 1-methoxymethyl pseudouridine, 5-methyl cytidine, 5-methoxy cytidine, 1-methyl-pseudouridine (N1-methyl-pseudo-UTP), and pseudouridine; preferably, the mRNA is chemically modified with 1-methyl-pseudouridine; preferably, uracil is replaced by 1-methyl-pseudouridine at a level of 70%, 80%, 85%, 90%, 95%, 99%, or 100%; most preferably, uracil is completely replaced by 1-methyl-pseudouridine, i.e., no natural uracil is used and 100% 1-methyl-pseudouridine is used.

In some embodiments, the aforementioned polynucleotide is a DNA or an RNA.

In some embodiments, the aforementioned polynucleotide is a coding RNA.

In some embodiments, the coding RNA is an mRNA, a self-replicating RNA, a circular RNA, or a replicon RNA, preferably an mRNA.

In some embodiments, the mRNA comprises at least one 3'-polyadenylic acid sequence, wherein the 3'-polyadenylic acid sequence comprises 30 to 200 adenosine nucleotides, and the 3'-terminal nucleotide is adenosine.

In some embodiments, the mRNA preferably comprises a 5'-cap structure, wherein the 5'-cap structure is preferably an m⁷G structure, a cap0 structure, a cap1 structure, a cap2 structure, a modified cap0 structure, or a modified cap1 structure, preferably a cap1 structure, and most preferably m7G(5')ppp(5')(2'OMeA)pG.

**In a third aspect**, the present application provides a composition, preferably an immunogenic composition comprising at least one polynucleotide according to the first aspect. Suitably, the composition may comprise at least one polynucleotide, e.g., at least one coding RNA, wherein the polynucleotide is complexed with one or more lipids, encapsulated in one or more lipids, or associated with one or more lipids, thereby forming a lipid nanoparticle.

In some embodiments, the present application relates to a rabies virus nucleic acid vaccine, wherein the vaccine carrier is a lipid nanoparticle (LNP) comprising an ionizable cationic lipid, a structural lipid, a helper lipid, and a surfactant; in some embodiments, the total molar content of the ionizable cationic lipid, the structural lipid, the helper lipid, and the surfactant is 100% by molar percentage (mol%).

In some embodiments, the lipid nanoparticle comprises 20-60 mol% ionizable cationic lipid, 25-55 mol% structural lipid, 5-25 mol% helper lipid, and 0.5-15 mol% surfactant. In some embodiments, the cationic lipid is selected from SM-102, ALC-0315, ALC-0519, Dlin-MC3-DMA (also known as MC3), DODMA, DLin-KC2-DMA, and DlinDMA, preferably SM-102; the structure of SM-102 (heptadecan-9-yl 8-((2-hydroxyethyl)(6-oxo-6-(undecyloxy)hexyl)amino)octanoate) is as follows:

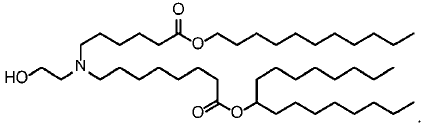

In some embodiments, the structural lipid includes cholesterol and cholesterol derivatives, preferably cholesterol.

In some embodiments, the helper lipid is selected from DSPC, DOPE, DOPC, DOPG, and DOPS, preferably DSPC.

In some embodiments, the surfactant is selected from PEG2000-DMG, PEG-DSPE, DTDA-PEG2000, and TPGS, preferably PEG2000-DMG.

In some embodiments, the lipid nanoparticle comprises 20-50 mol% ionizable cationic lipid. For example, the lipid nanoparticle may comprise 41, 42, 43, 44, 45, 46, 47, 48, 49, or 50 mol% ionizable cationic lipid.

In some other embodiments, the lipid nanoparticle comprises 50-60 mol% ionizable cationic lipid. For example, the lipid nanoparticle may comprise 51, 52, 53, 54, 55, 56, 57, 58, 59, or 60 mol% ionizable cationic lipid.

In some embodiments, the lipid nanoparticle comprises 5-25 mol% DSPC; for example, the lipid nanoparticle may comprise 5, 6, 7, 8, 9, 10, 11, 12, 13, 14, or 15 mol% DSPC. In some embodiments, the lipid nanoparticle comprises 25-55 mol% cholesterol. For example, the lipid nanoparticle may comprise 30, 31, 32, 33, 34, 35, 36, 37, 38, 38.5, 39, 39.5, or 40 mol% cholesterol.

In some embodiments, the lipid nanoparticle comprises 0.5-15 mol% DMG-PEG. For example, the lipid nanoparticle may comprise 0.5, 1, 1.5, or 2 mol% DMG-PEG.

In some embodiments, the lipid nanoparticle comprises 50 mol% ionizable cationic lipid, 10 mol% DSPC, 38.5 mol% cholesterol, and 1.5 mol% DMG-PEG.

In some embodiments, the lipid nanoparticle comprises 50 mol% SM-102, 10 mol% DSPC, 38.5 mol% cholesterol, and 1.5 mol% PEG2000-DMG.

In some embodiments, the lipid nanoparticle of the present application has a nitrogen-to-phosphorus ratio (N:P ratio) of about 2:1 to about 30:1.

In some embodiments, the lipid nanoparticle of the present application has an N:P ratio of about 6:1.

In some embodiments, the lipid nanoparticle of the present application has an N:P ratio of about 3:1.

In some embodiments, the lipid nanoparticle of the present application comprises an ionizable cationic lipid component and an RNA in a wt/wt ratio of about 10:1 to about 100:1.

In some embodiments, the lipid nanoparticle of the present application comprises an ionizable cationic lipid component and an RNA in a wt/wt ratio of about 20:1.

In some embodiments, the lipid nanoparticle of the present application comprises an ionizable cationic lipid component and an RNA in a wt/wt ratio of about 10:1. In some embodiments, the lipid nanoparticle of the present application has an average diameter of about 50 nm to about 150 nm.

In some embodiments, the lipid nanoparticle of the present application has an average diameter of about 70 nm to about 120 nm, preferably 100-120 nm, and most preferably 100 nm.

In some embodiments, the rabies virus nucleic acid vaccine of the present application further comprises: a buffer component and a cryoprotectant.

In some embodiments, the buffer may be selected from: a citrate buffer, an acetate buffer, a phosphate buffer, ammonium chloride, calcium carbonate, calcium chloride, calcium citrate, calcium gluconate, calcium glycerophosphate, calcium lactate, calcium lactobionate, propionic acid, calcium levulinate, valeric acid, dicalcium phosphate, phosphoric acid, tricalcium phosphate, potassium acetate, potassium chloride, potassium gluconate, a potassium mixture, dipotassium phosphate, tromethamine, dibasic potassium phosphate, a potassium phosphate mixture, sodium acetate, sodium bicarbonate, sodium chloride, sodium citrate, sodium lactate, disodium hydrogen phosphate, sodium dihydrogen phosphate, sodium phosphate, magnesium hydroxide, aluminum hydroxide, alginic acid, citric acid, polyethylene glycol, sodium benzoate, leucine, magnesium lauryl sulfate, sodium dodecyl sulfate, and combinations thereof.

In some embodiments, the cryoprotectant may be selected from substances such as sugars/polyols, polymers, surfactants, amino acids, and salts, wherein the sugars may be selected from: lactose, sucrose, trehalose, galactose, and the like.

In some embodiments, the amount of the cryoprotectant is 1 to 50% w/w, such as from 2 to 50% w/w, or from 4 to 45% w/w, or from 6 to 12% w/w, or preferably from 6 to 10% w/w, or most preferably from 7 to 9% w/w.

In some embodiments, the pharmaceutical composition of the present application comprises the aforementioned lipid nanoparticle composition and an external phase buffer.

In some embodiments, the aqueous phase buffer includes: tromethamine, sodium acetate, and sucrose, with a pH of 7-8.

In some embodiments, the content of tromethamine is selected from 10-30 mmol/L, preferably 15-25 mmol/L, preferably 15 mmol/L, 15.5 mmol/L, 16 mmol/L, 16.5 mmol/L, 17 mmol/L, 17.5 mmol/L, 18 mmol/L, 18.5 mmol/L, 19 mmol/L, 19.5 mmol/L, 20 mmol/L, 20.5 mmol/L, 21 mmol/L, 21.5 mmol/L, 22 mmol/L, 22.5 mmol/L, 23 mmol/L, 23.5 mmol/L, 24 mmol/L, 24.5 mmol/L, and 25 mmol/L, and most preferably 20 mmol/L.

In some embodiments, the content of sodium acetate is selected from 0-20 mmol/L, preferably 5-11 mmol/L, preferably 5 mmol/L, 5.5 mmol/L, 6 mmol/L, 6.5 mmol/L, 7 mmol/L, 7.5 mmol/L, 8 mmol/L, 8.5 mmol/L, 9 mmol/L, 9.5 mmol/L, 10 mmol/L, 10.5 mmol/L, 10.6 mmol/L, 10.7 mmol/L, 10.8 mmol/L, 10.9 mmol/L, 11 mmol/L, 11.5 mmol/L, 12 mmol/L, 12.5 mmol/L, and 13 mmol/L, and most preferably 10.7 mmol/L. In some embodiments, the content (w/w) of sucrose is selected from 5%-15%, preferably 7.5%-10%, more preferably 7.6%, 7.7%, 7.8%, 7.9%, 8.0%, 8.1%, 8.2%, 8.3%, 8.4%, 8.5%, 9%, 9.5%, or 10%, and most preferably 8.7%.

In some embodiments, the pharmaceutical composition of the present application comprises an mRNA which comprises the sequence set forth in SEQ ID NO. 1, a lipid nanoparticle composition, 20 mmol/L tromethamine, 10.7 mmol/L sodium acetate, and 8.7% sucrose, with a pH of 7.0-8.0, wherein the lipid nanoparticle comprises 50 mol% SM-102, 10 mol% DSPC, 38.5 mol% cholesterol, and 1.5 mol% PEG2000-DMG, at N:P of 6.

**In a fourth aspect**, the present application provides a rabies virus nucleic acid vaccine comprising a nucleic acid sequence encoding a rabies virus protein or a fragment thereof.

In some embodiments, the rabies virus protein includes, but is not limited to: glycoprotein (G protein), nucleocapsidprotein (N protein), phosphoprotein (P protein), matrix protein (M protein), and transcriptase protein.

In some embodiments, the nucleic acid vaccine comprises the sequence set forth in any one of SEQ ID NOs. 1-3 or a nucleotide sequence having at least 75%, at least 80%, at least 90%, at least 95%, at least 96%, at least 97%, at least 98%, at least 99%, or 100% identity to SEQ ID NOs. 1-3.

In some embodiments, the nucleic acid vaccine comprises a nucleotide sequence encoding SEQ ID NO. 4 or having at least 75%, at least 80%, at least 90%, at least 95%, at least 96%, at least 97%, at least 98%, at least 99%, or 100% identity thereto.

In some embodiments, the nucleic acid includes, but is not limited to, a DNA and an RNA, preferably an mRNA.

In some embodiments, the route of administration of the vaccine includes intravenous injection, intramuscular injection, or subcutaneous injection, preferably intramuscular injection.

In some embodiments, the dosage form of the vaccine may be selected from a lyophilized powder injection, a liquid dosage form for injection, and an inhalation formulation.

**In a fifth aspect**, the present application relates to a preparation method for a nucleic acid vaccine, comprising mixing a vaccine carrier with an mRNA to obtain the nucleic acid vaccine.

In some embodiments, the vaccine carrier is a cationic lipid nanoparticle, and the preparation method comprises the following specific steps:
(1) dissolving a protonatable cationic lipid, a structural lipid, a helper lipid, and a surfactant in an organic solution according to the formula proportion to obtain an organic phase;
(2) dissolving the optimized mRNA in a citrate buffer or a sodium acetate solution to obtain an aqueous phase; and
(3) uniformly mixing the organic phase in step (1) and the aqueous phase in step (2) to produce a mixed solution, so as to obtain a rabies virus vaccine.

In some embodiments, the organic solution includes absolute ethanol.

In some embodiments, the total concentration of the protonatable cationic lipid, the structural lipid, the helper lipid, and the surfactant in the organic phase is 10 to 15 mg/mL.

In some embodiments, the concentration of the mRNA is 0.01 to 1 mg/mL, preferably 0.1 to 0.2 mg/mL.

In some embodiments, the volume ratio of the organic phase to the aqueous phase is 1:(2 to 4).

In some embodiments, uniformly mixing is performed by adopting a microfluidic device, with a flow rate controlled to be ≥ 12 mL/min.

**In a sixth aspect**, the present application further relates to use of the mRNA or the composition in the preparation of a vaccine.

In some embodiments, the vaccine includes a combination vaccine and a multivalent vaccine.

In some embodiments, the vaccine is a rabies virus mRNA vaccine.

**In a seventh aspect**, the present disclosure further relates to a method for pre-exposure or post-exposure immunization, comprising administering to a subject the composition according to the third aspect, which is able to effectively induce an immune response against a rabies virus antigen in the subject.

**In an eighth aspect**, the present disclosure further relates to a nucleic acid molecule corresponding to the mRNA (a DNA sequence encoding the mRNA).

In some embodiments, the nucleotide sequence of the nucleic acid molecule is set forth in SEQ ID NO. 10.

In one embodiment, the present disclosure further relates to a biomaterial expressing the nucleic acid molecule.

In one embodiment, the biomaterial comprises: an expression cassette, recombinant vector, recombinant plasmid, transgenic cell line, and the like comprising the nucleic acid molecule.

In one embodiment, the recombinant vector is preferably a pVAX.1 vector or a pcDNA3.1 vector.

**According to the first aspect to the eighth aspect**, a strain of the rabies virus comprises, but is not limited to: a Pasteur strain (PAS), a Flury strain, a SAD strain, an aG strain, a CTN strain, SC16 (CSC1016D), GD1 (GDZAQ45), NM3 (CNM1103C), QH2 (CQH1202D), LY (CNX8601), YN3 (CYN1009D), XZ17 (CXZ1704H), and derivative strains thereof, preferably a CTN strain, and most preferably a CTN-1 strain. Compared with the prior art, the anti-rabies virus mRNA vaccine of the present disclosure has the following characteristics:
1. Comprehensively considering the prevalence of rabies virus in China, the CTN-1 strain is selected as the starting strain for the development of mRNA vaccines.
2. In order to achieve the optimal expression effect and stability of the mRNA sequence *in vivo*, the nucleotide coding sequence of the G protein of the CTN-1 strain is optimized, including: adjusting the codon preference during expression in humans, adjusting the frequency of use of common codons, increasing the GC content of the sequence, etc.; and the optimized nucleotide sequences are screened to finally determine the optimal sequence; the nucleotide sequence obtained by the present application results in a more stable transcribed mRNA structure and higher translation efficiency of the target protein in mammals and humans, is capable of effectively producing neutralizing antibodies, elicit the body to generate humoral immunity and cellular immunity, and can generate memory B cells and T cells; and the nucleotide sequence can induce animals to generate protective effects against various strains of rabies virus and mutant strains thereof with a relatively small dose.
3. In the present disclosure, the mRNA is delivered using the LNP method, and the stability of the mRNA in the LNP is good; after storage at a relatively high temperature of 5±3 °C for 12 weeks, each test item is maintained at a relatively high level; similarly, at -20 °C, each test index is also very good, and the vaccine efficacy is not significantly reduced after storage, which can meet the storage requirements for the large-scale use of mRNA vaccines.

### Definitions

Unless defined otherwise, all technical and scientific terms used herein have the same meaning as commonly understood by those of ordinary skill in the art. For the definitions and terminology in the art, professionals can refer specifically to Current Protocols in Molecular Biology (Ausubel). Abbreviations for amino acid residues refer to standard 3-letter and/or 1-letter codes used in the art to denote one of the 20 commonly used L-amino acids.

Although the numerical ranges and parameter approximations are shown in the broad scope of the present application, the numerical values shown in the specific examples are recorded as accurately as possible. However, any numerical values inherently contain certain errors necessarily resulting from the standard deviations found in their respective measurements. In addition, all ranges disclosed herein should be understood to encompass any and all subranges subsumed therein. For example, a recorded range of "1 to 10" should be considered to include any and all subranges between the minimum value of 1 and the maximum value of 10 (including the endpoints); that is, all subranges starting with a minimum value of 1 or greater, such as 1 to 6.1, and all subranges ending with a maximum value of 10 or less, such as 5.5 to 10. Additionally, any reference referred to as "incorporated herein" should be understood as being incorporated in its entirety.

It should be understood by those skilled in the art that many different polynucleotides may encode the same polypeptide due to the degeneracy of the genetic code. It should also be understood that skilled persons may, using conventional techniques, make nucleotide substitutions that do not affect the polypeptide sequence encoded by the nucleic acid molecule to reflect the codon usage of any particular host organism in which the polypeptide is expressed. Thus, unless otherwise stated, "polynucleotides encoding the protein or immunogenic fragment of the present application" include all polynucleotide sequences that are degenerate to each other and encode the same amino acid sequence.

### Antigen

As used herein, an antigen is a protein that is capable of inducing an immune response (e.g., causing the immune system to produce an antibody against the antigen). Herein, unless otherwise stated, the use of the term "antigen" includes immunogenic proteins and immunogenic fragments (immunogenic fragments that induce or are capable of inducing an immune response against at least one rabies virus). It should be understood that the term "protein" includes peptides, and the term "antigen" includes antigen fragments. Other molecules may also be antigenic, such as bacterial polysaccharides or a combination of proteins and polysaccharide structures. Viral vaccine antigens described herein include viral proteins, viral protein fragments, and engineered and/or mutated proteins derived from the rabies virus.

In addition, the term "antigen" as used herein will be recognized and understood by those of ordinary skill in the art, and means a substance that can be recognized by the immune system, preferably the adaptive immune system, and is capable of triggering an antigen-specific immune response, for example, by forming antibodies and/or antigen-specific T cells as part of the adaptive immune response. The antigen may be or may comprise a peptide or protein, which may be presented by MHC to a T cell. Also included are fragments, variants, and derivatives derived from, e.g., peptides or proteins.

Antigen peptide or protein: The term "antigen peptide or protein" or "immunogenic peptide or protein" will be recognized and understood by those of ordinary skill in the art, and means a peptide or protein derived from a protein (an antigenic or immunogenic protein) that stimulates the adaptive immune system of the body to provide an adaptive immune response. Thus, the antigenic/immunogenic peptide or protein comprises the protein from which it is derived.

As those skilled in the art will recognize and be familiar with, protein fragments, functional protein domains, and homologous proteins are also considered to be within the scope of the rabies virus antigen of interest, such as any protein fragment of the rabies virus or a mutant strain thereof, provided that the fragment has immunogenicity and confers a protective immune response against the rabies virus; in addition to the identical but truncated variant of the reference protein, in some embodiments, the antigen comprises 2, 3, 4, 5, 6, 7, 8, 9, 10, or more mutations, and the length of the antigen/antigen polypeptide can range from about 4, 6, or 8 amino acids to the full-length protein.

### Epitope

Epitope: The term "epitope" (also referred to in the art as "antigenic determinant") as used herein will be recognized and understood by those of ordinary skill in the art to mean T cell epitopes and B cell epitopes. T cell epitopes or parts of antigen peptides or proteins may comprise fragments preferably having a length of about 6 to about 20 or even more amino acids, e.g., fragments that are processed and presented by MHC class I molecules, preferably having a length of about 8 to about 10 amino acids, e.g., 8, 9, or 10 amino acids (or 11 or 12 amino acids), or fragments that are processed and presented by MHC class II molecules, preferably having a length of about 13 to about 20 or even more amino acids. These fragments are generally recognized by T cells in the form of a complex consisting of the peptide fragment and an MHC molecule, i.e., these fragments are generally not recognized in their natural form. B cell epitopes are generally fragments located on the outer surface of (natural) protein or peptide antigens, preferably having 5 to 15 amino acids, more preferably having 5 to 12 amino acids, even more preferably having 6 to 9 amino acids, which may be recognized by antibodies, i.e., in their natural form. Such epitopes of proteins or peptides may also be selected from any of the variants of such proteins or peptides referred to herein. Herein, an epitope may be a conformational or discontinuous epitope consisting of fragments of the protein or peptide as defined herein that are discontinuous in the amino acid sequence of the protein or peptide as defined herein, but are brought together in the three-dimensional structure, or may be a continuous or linear epitope consisting of a single polypeptide chain.

### Nucleic acid

The term "nucleic acid" or "nucleic acid molecule" will be recognized and understood by those of ordinary skill in the art. As used herein, the term "nucleic acid" or "nucleic acid molecule" preferably refers to a DNA (molecule) or an RNA (molecule). It is preferably used synonymously with the term polynucleotide. Preferably, the nucleic acid or nucleic acid molecule is a polymer comprising or consisting of nucleotide monomers covalently linked to each other by phosphodiester bonds in a sugar/phosphate backbone. The term "nucleic acid molecule" also includes modified nucleic acid molecules, e.g., DNA or RNA molecules with base modifications, sugar modifications, or backbone modifications as defined herein.

Specifically, the composition disclosed in the present application comprises (at least one) RNA having an open reading frame (ORF) encoding a rabies virus antigen. In some embodiments, the RNA is a messenger RNA (mRNA).

In some embodiments, the nucleic acid comprises at least one heterologous untranslated region (UTR). The term "untranslated region" or "UTR" or "UTR element" will be recognized and understood by those of ordinary skill in the art to mean a portion of a nucleic acid molecule, which is generally located at the 5' or 3' end of a coding sequence. The portion located at the 5' end is referred to as a 5' UTR, and the portion located at the 3' end is referred to as a 3' UTR. In general, the UTR is not translated into a protein; the UTR may be a portion of a nucleic acid, such as a DNA or an RNA. The UTR may comprise elements for controlling gene expression, which are also known as regulatory elements. Such regulatory elements may be ribosome-binding sites, miRNA-binding sites, etc.; the RNA (e.g., mRNA) may further comprise a 5' UTR, a 3' UTR, 3'-polyadenylic acid, and/or a 5' cap analog.

In some embodiments, the 5' UTR is a heterologous UTR, i.e., a UTR found in nature that is associated with different ORFs; in another embodiment, the 5' UTR is a synthetic UTR; the 5' UTR is a region of an mRNA located upstream (5') of a start codon (the first codon of an mRNA transcript translated by a ribosome). The 5' UTR does not encode a protein. A natural 5' UTR is characterized by playing a role in translation initiation and has features like a Kozak sequence, the Kozak sequence having the consensus sequence CCR(A/G)CCAUGG; exemplary 5' UTRs also include 5' UTRs of *Xenopus laevis* or human α-globin or β-globin, human cytochrome b-245a polypeptide, hydroxysteroid (17b) dehydrogenase, tobacco etch virus, alpha-1-globin, and the like. In some embodiments, the 3' UTR may be heterologous or synthetic; for example, the globin UTR includes a *Xenopus laevis* β-globin UTR and a human β-globin UTR; other 3' UTRs may also be 3' UTRs of CYBA (cytochrome b-245 alpha chain), rabbit β-globin, hepatitis B virus (HBV), and α-globin, and a VEEV (Venezuelan equine encephalitis virus) virus 3' UTR sequence. In some embodiments, rps9 (ribosomal protein S9) 3' UTR, and 3' UTRs of FIG4 (FIG4 phosphoinositide 5-phosphatase), gp130, DH143, human albumin hHBB (human hemoglobin subunit beta), and HBA1 (human hemoglobin subunit alpha 1) may also be used.

In some embodiments, the 3'-polyadenylic acid is also referred to as a poly-A tail. The poly(A) tail is an mRNA region that is located downstream, e.g., directly downstream (i.e., 3'), of a 3' UTR and contains a plurality of consecutive adenosine monophosphates. The poly(A) tail may contain 10 to 300 adenosine monophosphates, or may contain 10, 20, 30, 40, 50, 60, 70, 80, 90, 100, 110, 120, 130, 140, 150, 160, 170, 180, 190, 210, 220, 230, 240, 250, 260, 270, 280, 290, or 300 adenosine monophosphates. In some preferred embodiments, the poly(A) tail contains 50-250 adenosine monophosphates, more preferably 50-100 adenosine monophosphates, and most preferably 100 adenosine monophosphates. In the relevant biological environment (e.g., in a cell or *in vivo*), the 3'-polyadenylic acid tail functions to protect the mRNA from enzymatic degradation, e.g., in the cytoplasm, and to facilitate transcription termination and/or export of the mRNA from the nucleus and translation.

In some embodiments, the RNA (e.g., mRNA) further comprises a 5' guanosine cap. The 5' guanosine cap is on a eukaryotic mRNA transcript. The 5' cap consists of an inverted 7-methylguanosine and is linked to the rest of the eukaryotic mRNA via a 5'-5' triphosphate bridge, i.e., the so-called cap0. The cap0 primarily serves as a quality control for correct mRNA processing and helps stabilize the eukaryotic mRNA. On the basis of cap0, the first nucleotide is subjected to 2'-OH methylation, which is known as cap1. In addition to cap0 and cap1, a further methylation modification may also be performed on the second nucleotide, which is known as cap2. In general, the 5'-cap may be synthesized by different synthetic routes for the 5' capped mRNA based on enzymatic, chemical, or chemoenzymatic methods.

In some embodiments, in *in vitro* transcription, a cap analog is directly added to an *in vitro* transcription (IVT) system, and the 5' cap analog includes, but is not limited to: m7Gppp(2'OMeA)pG, m7GpppApA, m7GpppApC, m7GpppApG, m7GpppApU, m7GpppCpA, m7GpppCpC, m7GpppCpG, m7GpppCpU, m7GpppGpA, m7GpppGpC, m7GpppGpG, m7GpppGpU, m7GpppUpA, m7GpppUpC, m7GpppUpG, m7GpppUpU, m7Gpppm6ApG, m7G3'OmepppApA, m7G3'OmepppApC, m7G3'OmepppApU, m7G3'OmepppApG, m7G3'OmepppCpA, m7G3'OmepppCpC, m7G3'OmepppCpG, m7G3'OmepppCpU, m7G3'OmepppUpA, m7G3'OmepppUpC, m7G3'OmepppUpG, m7G3'OmepppUpU, m7G3'OmePppA2'OmepG, m7G3'OmepppA2'OmepC, m7G3'OmepppA2'OmepU, m7G3'OmepppA2'OmepA, m7G3'OmepppC2'OmepA, m7G3'OmepppC2'OmepU, m7G3'OmepppC2'OmepG, m7G3'OmepppC2'OmepC, m7G3'OmepppG2'OmepA, m7G3'OmepppG2'OmepU, m7G3'OmepppG2'OmepG, m7G3'OmepppG2'OmepC, m7G3'OmepppU2'OmepA, m7G3'OmepppU2'OmepU, m7G3'OmepppU2'OmepG, m7G3'OmepppU2'OmepC, and the like.

In some embodiments, the cap analog may also be of other structures, such as a tetramer, a pentamer, a hexamer, a heptamer, an octamer, a nonamer, a decamer, or the like. The specific sequence may be determined according to the condition of the template.

It should also be understood that the rabies virus vaccine disclosed in the present application may comprise any 5' untranslated region (UTR) and/or any 3' untranslated region (UTR). Nucleic acids comprise a polymer of nucleotides (nucleotide monomers). Thus, nucleic acids are also referred to as polynucleotides. Nucleic acids may be or may include, for example, deoxyribonucleic acids (DNAs), ribonucleic acids (RNAs), threose nucleic acids (TNAs), glycol nucleic acids (GNAs), peptide nucleic acids (PNAs), locked nucleic acids (LNAs), ethylene nucleic acids (ENA), cyclohexenyl nucleic acids (CeNA), and/or chimeras, and/or combinations thereof.

Messenger RNA (mRNA) is any RNA that encodes a (at least one) protein (a naturally-occurring, non-naturally-occurring, or modified polymer of amino acids) and can be translated *in vitro*, *in vivo*, *in situ*, or *ex vivo* to produce the encoded protein. The skilled person will appreciate that, unless otherwise stated, nucleic acid sequences set forth in the present application may recite "T" in a representative DNA sequence, but where the sequence represents RNA (e.g., mRNA), the "T" would be replaced by "U". Thus, for any DNA disclosed and identified herein by a particular sequence identification number, a corresponding RNA (e.g., mRNA) sequence complementary to the DNA is also disclosed, wherein each "T" of the DNA sequence is substituted with "U".

### Open reading frame

An open reading frame (ORF) is a contiguous stretch of DNA or RNA that begins with a start codon (e.g., methionine (ATG or AUG)) and ends with a stop codon (e.g., TAA, TAG or TGA, or UAA, UAG, or UGA). Generally, an ORF encodes a protein. It should be understood that the sequences disclosed herein may further comprise additional elements, e.g., 5' and 3' UTRs, but that those elements, unlike the ORF, are not necessarily present in the RNA polynucleotide disclosed in the present patent.

In some embodiments, the composition comprises an RNA (e.g., mRNA) comprising a nucleotide sequence having at least 80%, at least 85%, at least 90%, at least 95%, at least 98%, or 100% identity to the nucleotide sequence of any one of SEQ ID NOs. 1-6.

In some embodiments, the open reading frame is preferably codon-optimized at least in part. Codon optimization is based on the discovery that translation efficiency can be determined by different frequencies of the occurrence of transfer RNA (tRNA) in cells. Thus, if an increased degree of so-called "rare codons" is present in the coding regions of the nucleic acids of the present application as defined herein, the translation of the respective modified nucleic acid sequences is less efficient than when codons encoding relatively "common" tRNAs are present. Those skilled in the art may be able to perform codon optimization on the sequence to be translated according to the characteristics of its *in vitro* expression system.

### Chemically modified or unmodified nucleotide

In some embodiments, the RNA (e.g., mRNA) is not chemically modified, but comprises standard ribonucleotides consisting of adenosine, guanosine, cytidine, and uridine. In some embodiments, the nucleotides and nucleosides disclosed in the present application comprise standard nucleoside residues, such as those present in the transcribed RNA (e.g., A, G, C, or U). In some embodiments, the nucleotides and nucleosides disclosed in the present application comprise standard deoxyribonucleosides, such as deoxyribonucleosides present in the DNA (e.g., dA, dG, dC, or dT);

In some embodiments, the composition disclosed in the present application comprises an RNA having an open reading frame encoding a rabies virus antigen, wherein the nucleic acid comprises nucleotides and/or nucleosides known in the art that may be standard (unmodified) or modified. In some embodiments, the nucleotides and nucleosides disclosed in the present application include modified nucleotides or nucleosides. Such modified nucleotides and nucleosides may be naturally occurring modified nucleotides and nucleosides or non-naturally occurring modified nucleotides and nucleosides. Such modifications may include modifications at the sugar, backbone, or nucleobase portion of nucleotide and/or nucleoside that are well known in the art.

In some embodiments, the modified nucleic acid base in the nucleic acid (e.g., an RNA nucleic acid, e.g., an mRNA nucleic acid) includes 1-methyl-pseudouridine, 1-ethyl-pseudouridine, 5-methoxy-uridine, 5-methyl-cytidine, and pseudouridine.

### In vitro transcription system (IVT)

*In vitro* transcription involves using a DNA as a template to mimic the *in vivo* transcription process in an *in vitro* cell-free system containing components such as an RNA polymerase and NTPs to generate an mRNA. In general, the capped RNA synthesized in the *in vitro* transcription reaction may be used for subsequent assays such as microinjection, *in vitro* translation, and transfection. The *in vitro* transcription system typically comprises a transcription buffer, nucleotide triphosphates (NTPs), an RNase inhibitor, and a polymerase. NTPs may be self-synthesized or selected from suppliers. NTPs may be natural or unnatural NTPs. Optional polymerases include, but are not limited to, bacteriophage RNA polymerase, e.g., T7 RNA polymerase, T3 RNA polymerase, SP6 RNA polymerase, and/or polymerase mutants thereof, for example, but not limited to, polymerases capable of incorporating modified nucleic acids and/or modified nucleotides, including chemically modified nucleic acids and/or nucleotides. Some embodiments exclude the use of DNase. In some embodiments, the RNA comprises a 5' guanosine cap.

In addition to the synthesis in the *in vitro* transcription system, chemical synthesis methods can also be used, including solid-phase chemical synthesis and liquid-phase chemical synthesis. For solid-phase chemical synthesis, all or part of the nucleic acids disclosed in the present application can be prepared by using solid-phase techniques. Solid-phase chemical synthesis of nucleic acids is an automated method in which molecules are immobilized on a solid support and are synthesized step by step in a reactant solution. Solid-phase synthesis can be used for site-specific introduction of chemical modifications in nucleic acid sequences. For liquid-phase chemical synthesis, the synthesis of the nucleic acids disclosed in the present application by sequential addition of monomer constructs can be performed in a liquid phase. In addition, the synthesis methods described above can also be used in combination, as each of the synthesis methods discussed above has its own advantages and limitations, and these methods can be tried in combination to overcome the limitations described above. Combinations of these methods are within the scope disclosed in the present application.

### Antigen variant

In some embodiments, the composition disclosed in the present application comprises an RNA encoding a rabies virus antigen variant. Antigen variants or other polypeptide variants refer to molecules whose amino acid sequences differ from the wild-type, natural, or reference sequence. Antigen/polypeptide variants may have substitutions, deletions, and/or insertions at certain positions within the amino acid sequence compared to the natural or reference sequence. Generally, the variant has at least 50% identity to the wild-type, natural, or reference sequence. In some embodiments, the variant has at least 80%, or at least 90%, 91%, 92%, 93%, 94%, 95%, 96%, 97%, 98%, or 99% identity to the wild-type, natural, or reference sequence.

Variant antigens/polypeptides encoded by nucleic acids in the content disclosed in the present application may contain amino acid changes that confer any of a variety of desirable properties, e.g., enhancing their immunogenicity, enhancing their expression, and/or improving their stability or PK/PD properties. Variant antigens/polypeptides can generally be prepared using conventional mutagenesis techniques and analyzed to determine whether they possess the desired properties as appropriate. Assays to determine expression levels and immunogenicity are well known in the art, and examples of such assays are set forth in the Examples section. Similarly, the PK/PD properties of protein variants can be measured using techniques recognized in the art, e.g., by determining the expression of an antigen in a vaccinated subject over time and/or by observing the persistence of the induced immune response. The stability of a protein encoded by a variant nucleic acid may be measured by determining the thermal stability or stability upon urea denaturation, or may be measured using computational prediction. Methods for such experiments and computational determination are known in the art.

### Identity

The term "identity" refers to a relationship between the sequences of two or more polypeptides (e.g., antigens) or polynucleotides (nucleic acids), as determined by comparing the sequences. The identity also refers to the degree of sequence relatedness between the sequences, as determined by the number of matches between strings of two or more amino acid residues or nucleic acid residues. The identity measures the percentage of identical matches between the smaller sequences of two or more sequences, where gap alignments (if any) are addressed by a particular mathematical model or computer program (e.g., an "algorithm"). Identity of related antigens or nucleic acids can be readily calculated by known methods. "Percent (%) identity" for a polypeptide or polynucleotide sequence is defined as the percentage of residues (amino acid residues or nucleic acid residues) in a candidate amino acid or nucleic acid sequence that are identical to the residues in a second sequence after aligning the sequences and introducing gaps, if necessary, to achieve the maximum percent identity. Methods and computer programs for alignment are well known in the art. It will be understood that the identity depends on the calculation of percent identity, but may vary in value due to gaps and penalties introduced in the calculation. Generally, a variant of a particular polynucleotide or polypeptide (e.g., antigen) has a sequence identity of 40%, 45%, 50%, 55%, 60%, 65%, 70%, 75%, 80%, 85%, 90%, 91%, 92%, 93%, 94%, 95%, 96%, 97%, 98%, 99%, or 100%, as determined by sequence alignment programs and parameters described herein for the particular reference polynucleotide or polypeptide and known to those skilled in the art.

### Lipid nanoparticle (LNP)

The RNA (e.g., mRNA) disclosed in the present application is formulated in a lipid nanoparticle (LNP). The lipid nanoparticle generally comprises an ionizable cationic lipid, a helper lipid, cholesterol, and a PEG lipid component, as well as a nucleic acid of interest. The lipid nanoparticle disclosed in the present application can be produced by using components, compositions, and methods generally known in the art.

### Multivalent vaccine

The composition provided herein may comprise an RNA or a plurality of RNAs encoding two or more antigens of identical or different species. In some embodiments, the composition comprises an RNA or a plurality of RNAs encoding two or more rabies virus antigens. In some embodiments, the RNA may encode 1, 2, 3, 4, 5, 6, 7, 8, 9, 10, 11, 12, or more rabies virus antigens.

Two or more different RNAs (e.g., mRNAs) encoding antigens may be formulated in the same lipid nanoparticle. In other embodiments, two or more different RNAs encoding antigens may be formulated in separate lipid nanoparticles (each RNA is formulated in a single lipid nanoparticle). Subsequently, the lipid nanoparticles may be combined and administered as a single vaccine composition (e.g., comprising a plurality of RNAs encoding a plurality of antigens), or may be administered separately.

### Combination vaccine

The composition provided herein may comprise an RNA or a plurality of RNAs encoding two or more antigens of identical or different viral strains. Also provided herein is a combination vaccine comprising an RNA encoding one or more rabies virus antigens and antigens of one or more different organisms. Thus, the vaccine disclosed in the present application may be a combination vaccine targeting one or more antigens of the same strain/species, or one or more antigens of different strains/species, such as an antigen that induces immunity to an organism found in the same geographic region where the risk of rabies virus infection is high, or an organism to which an individual may be exposed when exposed to the rabies virus.

### Pharmaceutical formulation

Provided herein are a composition (e.g., pharmaceutical composition), a method, a kit, and a reagent for preventing or treating rabies virus, e.g., in humans and other mammals. The compositions provided herein may be used as a therapeutic agent or prophylactic agent. They can be used in medicaments for the prevention and/or treatment of a rabies virus infection.

### Pharmaceutical composition

The term "pharmaceutical composition" refers to a combination of an active agent with an inert or active carrier, making the composition particularly suitable for diagnostic or therapeutic use *in vivo* or *in vitro*. "Pharmaceutically acceptable carrier" does not cause undesirable physiological effects upon or after administration to a subject. The carrier in a pharmaceutical composition must be "acceptable" in the sense that it is compatible with the active ingredient and capable of stabilizing the active ingredient. One or more solubilizers may be used as pharmaceutical carriers for delivery of the active agent. Examples of pharmaceutically acceptable carriers include, but are not limited to, biocompatible carriers, adjuvants, additives, and diluents, to obtain compositions that may be used as dosage forms. Examples of other carriers include colloidal silica, magnesium stearate, cellulose, and sodium dodecyl sulfate. Other suitable pharmaceutical carriers and diluents, as well as pharmaceutical necessities for them, are described in Remington's Pharmaceutical Sciences.

### Sequence information

Related sequences involved in the present application are as follows:
**SEQ ID NO. 1** 
GGGAGACCCAAGCUGGCUAGCGUUUAAACUUAAGCUUGGUACCGAGCUCGGAUCC
ACUAGUCCAGUGUGGUGGAAUUCAGAAUAAACUAGUAUUCUUCUGGUCCCCACAG
ACUCAGAGAGAACCCGCCACCAUGAUCCCUCAGGCCCUGCUGUUCGUGCCCCUGCU
GGUGUUUCCUCUGUGCUUCGGCAAGUUCCCAAUCUACACCAUCCCAGAUAAGCUGG
GACCUUGGAGCCCUAUCGACAUUCACCACCUGAGUUGUCCUAACAACCUGGUGGUC
GAGGACGAGGGCUGUACCAAUCUGAGCGGCUUCUCCUACAUGGAACUGAAGGUGG
GCUACAUCAGCGCCAUCAAGGUGAACGGCUUCACUUGCACCGGCGUGGUGACCGAG
GCCGAGACAUACACAAAUUUCGUUGGCUACGUGACCACCACCUUCAAGAGAAAGC
ACUUCCGCCCCACCCCUGACGCCUGUCGGAGCGCUUACAACUGGAAGAUGGCCGGC
GAUCCUAGAUACGAGGAAAGUCUGCACAACCCCUAUCCUGAUUACCACUGGCUGA
GAACCGUGAAGACCACAAAGGAAAGCGUGGUGAUCAUUAGCCCUAGCGUGGCCGA
UCUGGACCCUUACGACAAGUCUCUCCAUAGCAGAGUGUUCCCUAGAGGCAAGUGC
AGCGGCAUAACAGUGAGCUCCGCCUACUGCAGCACCAAUCACGACUACACCAUCUG
GAUGCCUGAGAACCCUAGACUGGGUACAUCUUGUGAUAUCUUCACAAACAGCAGA
GGCAAACGGGCUUCUAAAGGCAGCAAGACCUGUGGCUUUGUGGACGAGCGGGGAC
UGUACAAAUCUCUGAAGGGCGCCUGCAAGCUGAAACUGUGCGGCGUGCUGGGCCU
CCGGCUGAUGGACGGCACCUGGGUCGCCAUCCAGACCAGCAACGAGACAAAGUGGU
GCCCCCCCGAUCAGCUGGUGAAUCUGCACGAUUUCCACAGCGACGAAAUCGAGCAU
CUGGUGGUGGAAGAACUGGUUAAAAAGCGGGAAGAGUGCCUGGAUGCUCUGGAAA
GCAUCAUGACAACCAAAUCCGUGAGCUUCCGGAGGCUGAGCCACCUGAGAAAGCU
GGUUCCCGGCUUCGGCAAGGCCUAUACCAUCUUUAACAAGACACUGAUGGAAGCC
GACGCCCACUACAAGAGCGUCCGGACCUGGAACGAGAUCAUCCCUAGCAAGGGCUG
CCUGAGAGUGGGCGGAAGAUGCCACCCCCACGUGAACGGCGUGUUUUUCAACGGC
AUCAUCCUGGGCCCUGACGGCCACGUGCUGAUCCCUGAGAUGCAGUCCAGCCUGCU
GCAGCAGCACAUGGAACUGCUGGAGAGCUCUGUGAUCCCCCUGAUGCACCCUCUGG
CCGACCCCAGCACAGUGUUUAAGGACGGCGACGAGGUGGAGGACUUCGUGGAAGU
GCACCUGCCUGACGUGCAUAAGCAGGUGAGCGGCGUGGACCUGGGACUGCCAAAC
UGGGGAAAAGACGUGCUGAUGGGCGCUGGCGUGCUGACCGCCCUGAUGCUGAUGA
UCUUCCUGAUGACAUGCUGCAGAAGAACCAACCGGGCCGAGAGCAUCCAACACAGC
CUGGGCGAGACCGGCAGAAAGGUGUCGGUCACCUCUCAGUCUGGAAGAGUGAUCA
GCUCUUGGGAGAGCUACAAGAGCGGAGGAGAAACCAAACUGUGAUAACUCGAGCU
GGUACUGCAUGCACGCAAUGCUAGCUGCCCCUUUCCCGUCCUGGGUACCCCGAGUC
UCCCCCGACCUCGGGUCCCAGGUAUGCUCCCACCUCCACCUGCCCCACUCACCACCU
CUGCUAGUUCCAGACACCUCCCAAGCACGCAGCAAUGCAGCUCAAAACGCUUAGCC
UAGCCACACCCCCACGGGAAACAGCAGUGAUUAACCUUUAGCAAUAAACGAAAGU
UUAACUAAGCUAUACUAACCCCAGGGUUGGUCAAUUUCGUGCCAGCCACACCCUGG
AGCUAGCAAAAAAAAAAAAAAAAAAAAAAAAAAAAAAGCAUAUGACUAAAAAAA
AAAAAAAAAAAAAAAAAAAAAAAAAAAAAAAAAAAAAAAAAAAAAAAAAAAAAAAA
 AAAAAAA
**SEQ ID NO. 2** 
GGGAGACCCAAGCUGGCUAGCGUUUAAACUUAAGCUUGGUACCGAGCUCGGAUCC
ACUAGUCCAGUGUGGUGGAAUUCAGAAUAAACUAGUAUUCUUCUGGUCCCCACAG
ACUCAGAGAGAACCCGCCACCAUGAUUCCCCAGGCACUGCUGUUCGUGCCGUUGCU
GGUUUUUCCACUGUGUUUUGGUAAGUUUCCCAUAUAUACCAUCCCCGAUAAACUG
GGCCCUUGGUCCCCCAUCGACAUCCACCACUUGAGCUGUCCCAACAAUCUGGUUGU
UGAGGACGAGGGCUGUACCAACCUCAGCGGCUUCAGUUAUAUGGAACUGAAGGUU
GGCUAUAUUAGCGCCAUCAAAGUGAAUGGCUUUACCUGCACCGGAGUGGUGACCG
AAGCCGAGACCUACACGAACUUUGUUGGUUACGUCACCACAACGUUUAAACGUAA
GCACUUCCGACCGACUCCAGAUGCCUGUAGGUCGGCCUACAAUUGGAAGAUGGCCG
GAGAUCCACGUUAUGAGGAAUCCCUCCAUAACCCAUACCCGGACUACCAUUGGCUG
AGGACGGUGAAGACAACGAAGGAGUCCGUGGUCAUAAUCUCGCCAUCCGUGGCCG
ACCUGGAUCCCUACGAUAAGAGCCUGCACUCCCGAGUGUUCCCACGAGGCAAGUGU
AGCGGUAUUACGGUCAGCAGCGCAUACUGUUCCACAAAUCACGAUUAUACUAUAU
GGAUGCCCGAGAACCCACGGCUGGGAACAAGUUGCGACAUUUUUACCAAUUCCAG
GGGAAAGCGGGCAUCGAAGGGAUCCAAGACAUGUGGCUUCGUCGAUGAACGGGGA
CUGUACAAGAGUCUUAAAGGCGCUUGUAAGCUGAAACUCUGUGGUGUUCUGGGAU
UGCGACUGAUGGAUGGAACCUGGGUGGCCAUUCAGACGAGCAAUGAGACCAAAUG
GUGCCCGCCCGAUCAAUUGGUGAAUCUGCAUGACUUUCACAGCGACGAGAUCGAG
CAUUUGGUGGUGGAGGAACUGGUGAAGAAGCGCGAAGAGUGCUUGGACGCCCUGG
AAUCGAUCAUGACCACUAAAUCGGUUUCGUUCCGCCGGCUUUCGCACCUCAGGAAG
UUGGUGCCGGGCUUCGGCAAGGCUUACACCAUUUUUAAUAAGACCCUGAUGGAGG
CUGACGCACACUACAAGUCCGUUAGGACUUGGAAUGAGAUCAUUCCAAGUAAGGG
CUGCUUGAGGGUAGGAGGUCGGUGCCAUCCGCAUGUGAAUGGUGUCUUCUUCAAU
GGCAUCAUUUUGGGACCGGAUGGCCAUGUGCUCAUCCCAGAGAUGCAGUCCUCCCU
CCUCCAACAGCACAUGGAACUGCUGGAGAGUUCGGUAAUUCCACUGAUGCAUCCCC
UGGCCGAUCCGAGCACAGUUUUUAAGGACGGUGAUGAGGUAGAAGACUUCGUCGA
GGUACAUUUGCCCGAUGUACACAAACAGGUUUCGGGAGUGGACCUUGGAUUGCCC
AACUGGGGCAAGGAUGUUUUGAUGGGAGCCGGAGUGCUCACCGCACUUAUGCUGA
UGAUUUUCCUGAUGACUUGCUGCCGCAGGACCAACAGGGCAGAGUCGAUUCAGCA
UAGCUUGGGCGAGACGGGUCGGAAAGUCUCCGUCACCAGUCAGUCCGGACGUGUG
AUCAGCUCCUGGGAGAGUUACAAAAGCGGCGGUGAAACCAAGCUGUGAUAACUCG
AGCUGGUACUGCAUGCACGCAAUGCUAGCUGCCCCUUUCCCGUCCUGGGUACCCCG
AGUCUCCCCCGACCUCGGGUCCCAGGUAUGCUCCCACCUCCACCUGCCCCACUCACC
ACCUCUGCUAGUUCCAGACACCUCCCAAGCACGCAGCAAUGCAGCUCAAAACGCUU
AGCCUAGCCACACCCCCACGGGAAACAGCAGUGAUUAACCUUUAGCAAUAAACGAA
AGUUUAACUAAGCUAUACUAACCCCAGGGUUGGUCAAUUUCGUGCCAGCCACACCC
UGGAGCUAGCAAAAAAAAAAAAAAAAAAAAAAAAAAAAAAGCAUAUGACUAAAAA
AAAAAAAAAAAAAAAAAAAAAAAAAAAAAAAAAAAAAAAAAAAAAAAAAAAAAAAA
AAAAAAAAAA
**SEQ ID NO. 3** 
GGGAGACCCAAGCUGGCUAGCGUUUAAACUUAAGCUUGGUACCGAGCUCGGAUCC
ACUAGUCCAGUGUGGUGGAAUUCAGAAUAAACUAGUAUUCUUCUGGUCCCCACAG
ACUCAGAGAGAACCCGCCACCAUGAUCCCCCAAGCUUUGCUGUUCGUGCCUCUCCU
GGUCUUUCCACUGUGCUUUGGGAAAUUCCCCAUUUACACCAUCCCAGAUAAGCUCG
GCCCUUGGUCUCCCAUCGACAUUCAUCACCUGUCUUGUCCUAACAAUUUGGUGGUG
GAGGACGAAGGAUGCACAAAUCUUAGCGGCUUCAGCUAUAUGGAAUUGAAAGUCG
GGUACAUAAGCGCCAUUAAGGUCAACGGUUUCACCUGCACGGGCGUGGUGACUGA
GGCGGAAACUUACACAAACUUUGUCGGCUAUGUGACCACGACAUUCAAGAGGAAA
CACUUCAGACCUACGCCAGAUGCUUGCAGGUCUGCUUAUAACUGGAAGAUGGCAG
GCGAUCCUAGGUAUGAGGAAUCUCUGCAUAACCCUUAUCCUGAUUAUCAUUGGCU
UCGCACGGUGAAAACCACCAAAGAGAGCGUGGUCAUUAUUUCACCAUCUGUGGCG
GAUCUGGACCCCUACGACAAAUCUCUGCACUCAAGAGUGUUUCCUCGGGGCAAAU
GCUCAGGUAUCACCGUUUCUAGUGCCUACUGCAGUACGAACCACGACUACACCAUU
UGGAUGCCCGAGAACCCUAGGUUGGGCACCUCUUGUGAUAUAUUCACUAACUCUA
GGGGAAAGCGGGCGUCUAAAGGGUCAAAGACCUGCGGAUUCGUUGACGAGAGGGG
ACUGUAUAAGAGCUUGAAGGGCGCCUGCAAGCUGAAACUGUGCGGGGUGCUUGGA
UUGCGCCUGAUGGAUGGCACCUGGGUGGCCAUUCAGACAAGCAAUGAAACCAAGU
GGUGUCCGCCCGAUCAGCUUGUGAACCUGCAUGACUUUCAUUCUGACGAGAUCGA
ACACCUCGUGGUCGAGGAGCUGGUGAAAAAGAGGGAAGAAUGCCUGGAUGCGCUC
GAGAGCAUUAUGACUACCAAAAGCGUGAGCUUCAGGCGAUUGAGCCACCUGCGCA
AGUUGGUGCCUGGCUUCGGGAAGGCUUAUACUAUUUUUAACAAGACUCUUAUGGA
GGCAGACGCUCAUUACAAGAGCGUCAGGACCUGGAAUGAAAUUAUUCCCAGCAAG
GGCUGCCUGAGAGUUGGGGGACGGUGUCACCCUCACGUUAACGGCGUGUUCUUCA
ACGGCAUCAUCCUGGGUCCCGACGGUCAUGUGCUGAUCCCCGAAAUGCAAAGUUCU
CUGCUCCAACAGCACAUGGAACUGCUGGAGUCCUCCGUGAUCCCACUGAUGCACCC
CCUGGCUGACCCCUCUACAGUGUUCAAAGAUGGGGACGAAGUCGAAGACUUUGUG
GAGGUCCACCUGCCCGACGUGCACAAACAGGUCUCAGGAGUGGAUUUGGGACUCCC
AAAUUGGGGCAAAGAUGUCCUGAUGGGUGCGGGGGUCCUGACAGCUCUGAUGCUC
AUGAUAUUCCUUAUGACAUGUUGCAGGCGCACCAACAGGGCCGAGAGUAUCCAGC
ACAGCUUGGGUGAGACUGGGCGGAAGGUGUCCGUUACAUCCCAGUCUGGCAGGGU
UAUCUCAUCCUGGGAGUCCUACAAGUCUGGAGGCGAGACUAAACUGUGAUAACUC
GAGCUGGUACUGCAUGCACGCAAUGCUAGCUGCCCCUUUCCCGUCCUGGGUACCCC
GAGUCUCCCCCGACCUCGGGUCCCAGGUAUGCUCCCACCUCCACCUGCCCCACUCA
CCACCUCUGCUAGUUCCAGACACCUCCCAAGCACGCAGCAAUGCAGCUCAAAACGC
UUAGCCUAGCCACACCCCCACGGGAAACAGCAGUGAUUAACCUUUAGCAAUAAACG
AAAGUUUAACUAAGCUAUACUAACCCCAGGGUUGGUCAAUUUCGUGCCAGCCACA
CCCUGGAGCUAGCAAAAAAAAAAAAAAAAAAAAAAAAAAAAAAAAAGCAUAUGACUAA
AAAAAAAAAAAAAAAAAAAAAAAAAAAAAAAAAAAAAAAAAAAAAAAAAAAAAAAA
AAAAAAAAAAAAAAA
 Note: In SEQ ID NOs. 1-3, U can be replaced by 1-methyl-pseudouridine at a level of 100%.
**SEQ ID NO. 4** 
MIPQALLFVPLLVFPLCFGKFPIYTIPDKLGPWSPIDIHHLSCPNNLVVEDEGCTNLSGFSYM
ELKVGYISAIKVNGFTCTGVVTEAETYTNFVGYVTTTFKRKHFRPTPDACRSAYNWKMA
 
GDPRYEESLHNPYPDYHWLRTVKTTKESVVIISPSVADLDPYDKSLHSRVFPRGKCSGITVS
SAYCSTNHDYTIWMPENPRLGTSCDIFTNSRGKRASKGSKTCGFVDERGLYKSLKGACKL
KLCGVLGLRLMDGTWVAIQTSNETKWCPPDQLVNLHDFHSDEIEHLVVEELVKKREECLD
ALESIMTTKSVSFRRLSHLRKLVPGFGKAYTIFNKTLMEADAHYKSVRTWNEIIPSKGCLR
VGGRCHPHVNGVFFNGIILGPDGHVLIPEMQSSLLQQHMELLESSVIPLMHPLADPSTVFK
DGDEVEDFVEVHLPDVHKQVSGVDLGLPNWGKDVLMGAGVLTALMLMIFLMTCCRRTN
RAESIQHSLGETGRKVSVTSQSGRVISSWESYKSGGETKL
**SEQ ID NO. 5** (KOZAK sequence)
   GCCACCATG
**SEQ ID NO. 6** (alpha-1-globin 5'-UTR) 
GGGAGACCCAAGCTGGCTAGCGTTTAAACTTAAGCTTGGTACCGAGCTCGGATCCACT
AGTCCAGTGTGGTGGAATTCAGAATAAACTAGTATTCTTCTGGTCCCCACAGACTCAGA
GAGAACCCGCCACC
**SEQ ID NO. 7** (3'-polyadenylic acid sequence) 
AAAAAAAAAAAAAAAAAAAAAAAAAAGCATATGACTAAAAAAAAAAA
AAAAAAAAAAAAAAAAAAAAAAAAAAAAAAAAAAAAAAAAAAAAAAA
A
**SEQ ID NO. 8 (gp130 3'-UTR)** 
CTCGAGCTGGTACTGCATGCACGCAATGCTAGCTGCCCCTTTCCCGTCCTGGGTACCCC
GAGTCTCCCCCGACCTCGGGTCCCAGGTATGCTCCCACCTCCACCTGCCCCACTCACC
ACCTCTGCTAGTTCCAGACACCTCC
**SEQ ID NO. 9 (DH143 3'-UTR)** 
CAAGCACGCAGCAATGCAGCTCAAAACGCTTAGCCTAGCCACACCCCCACGGGAAAC
AGCAGTGATTAACCTTTAGCAATAAACGAAAGTTTAACTAAGCTATACTAACCCCAGGG
TTGGTCAATTTCGTGCCAGCCAC
**SEQ ID NO. 10 (DNA sequence corresponding to sequence 1)**

GGGAGACCCAAGCTGGCTAGCGTTTAAACTTAAGCTTGGTACCGAGCTCGGATCCACT
AGTCCAGTGTGGTGGAATTCAGAATAAACTAGTATTCTTCTGGTCCCCACAGACTCAGA
GAGAACCCGCCACCATGATCCCTCAGGCCCTGCTGTTCGTGCCCCTGCTGGTGTTTCCT
CTGTGCTTCGGCAAGTTCCCAATCTACACCATCCCAGATAAGCTGGGACCTTGGAGCC
CTATCGACATTCACCACCTGAGTTGTCCTAACAACCTGGTGGTCGAGGACGAGGGCTG
TACCAATCTGAGCGGCTTCTCCTACATGGAACTGAAGGTGGGCTACATCAGCGCCATCA
AGGTGAACGGCTTCACTTGCACCGGCGTGGTGACCGAGGCCGAGACATACACAAATTT
CGTTGGCTACGTGACCACCACCTTCAAGAGAAAGCACTTCCGCCCCACCCCTGACGCC
TGTCGGAGCGCTTACAACTGGAAGATGGCCGGCGATCCTAGATACGAGGAAAGTCTGC
ACAACCCCTATCCTGATTACCACTGGCTGAGAACCGTGAAGACCACAAAGGAAAGCG
TGGTGATCATTAGCCCTAGCGTGGCCGATCTGGACCCTTACGACAAGTCTCTCCATAGC
AGAGTGTTCCCTAGAGGCAAGTGCAGCGGCATAACAGTGAGCTCCGCCTACTGCAGCA

CCAATCACGACTACACCATCTGGATGCCTGAGAACCCTAGACTGGGTACATCTTGTGAT
ATCTTCACAAACAGCAGAGGCAAACGGGCTTCTAAAGGCAGCAAGACCTGTGGCTTT
GTGGACGAGCGGGGACTGTACAAATCTCTGAAGGGCGCCTGCAAGCTGAAACTGTGC
GGCGTGCTGGGCCTCCGGCTGATGGACGGCACCTGGGTCGCCATCCAGACCAGCAAC
GAGACAAAGTGGTGCCCCCCCGATCAGCTGGTGAATCTGCACGATTTCCACAGCGACG
AAATCGAGCATCTGGTGGTGGAAGAACTGGTTAAAAAGCGGGAAGAGTGCCTGGATG
CTCTGGAAAGCATCATGACAACCAAATCCGTGAGCTTCCGGAGGCTGAGCCACCTGAG
AAAGCTGGTTCCCGGCTTCGGCAAGGCCTATACCATCTTTAACAAGACACTGATGGAA
GCCGACGCCCACTACAAGAGCGTCCGGACCTGGAACGAGATCATCCCTAGCAAGGGC
TGCCTGAGAGTGGGCGGAAGATGCCACCCCCACGTGAACGGCGTGTTTTTCAACGGC
ATCATCCTGGGCCCTGACGGCCACGTGCTGATCCCTGAGATGCAGTCCAGCCTGCTGC
AGCAGCACATGGAACTGCTGGAGAGCTCTGTGATCCCCCTGATGCACCCTCTGGCCGA
CCCCAGCACAGTGTTTAAGGACGGCGACGAGGTGGAGGACTTCGTGGAAGTGCACCT
GCCTGACGTGCATAAGCAGGTGAGCGGCGTGGACCTGGGACTGCCAAACTGGGGAAA
AGACGTGCTGATGGGCGCTGGCGTGCTGACCGCCCTGATGCTGATGATCTTCCTGATG
ACATGCTGCAGAAGAACCAACCGGGCCGAGAGCATCCAACACAGCCTGGGCGAGACC
GGCAGAAAGGTGTCGGTCACCTCTCAGTCTGGAAGAGTGATCAGCTCTTGGGAGAGC
TACAAGAGCGGAGGAGAAACCAAACTGTGATAACTCGAGCTGGTACTGCATGCACGC
AATGCTAGCTGCCCCTTTCCCGTCCTGGGTACCCCGAGTCTCCCCCGACCTCGGGTCCC
AGGTATGCTCCCACCTCCACCTGCCCCACTCACCACCTCTGCTAGTTCCAGACACCTCC
CAAGCACGCAGCAATGCAGCTCAAAACGCTTAGCCTAGCCACACCCCCACGGGAAAC
AGCAGTGATTAACCTTTAGCAATAAACGAAAGTTTAACTAAGCTATACTAACCCCAGGG
TTGGTCAATTTCGTGCCAGCCACACCCTGGAGCTAGCAAAAAAAAAAAAAAAAAAAA
AAAAAAAAAAGCATATGACTAAAAAAAAAAAAAAAAAAAAAAAAAAAAAAAAAAAA
AAAAAAAAAAAAAAAAAAAAAAAAAAAAAAAAAAA

### BRIEF DESCRIPTION OF THE DRAWINGS

FIG. 1 shows the purity of an mRNA encoding G protein analyzed by Bioanalyzer.
FIG. 2 shows the results of the LNP formula optimization experiment.
FIG. 3 shows the morphology of the complex of G protein mRNA and lipid nanoparticle (LNP) by transmission electron microscopy.
FIG. 4 shows the expression of the G protein in host cells as verified by the western blotting assay.
FIG. 5 shows the expression of the antigen protein of interest in cells as detected by an FACS flow cytometer.
FIG. 6 shows the detection of neutralizing antibodies in mice after a single immunization.
FIG. 7 shows the detection of neutralizing antibodies in mice immunized by different immunization procedures.
FIG. 8 shows the test results of neutralizing antibodies in cynomolgus monkeys.
FIG. 9 shows the hepatotoxicity results of the rabies mRNA vaccine.
FIG. 10 shows the concentration results of long-acting neutralizing antibodies for the rabies mRNA vaccine.
FIG. 11 shows the test results of neutralizing activity of the rabies mRNA vaccine against predominant strains in China.

### DETAILED DESCRIPTION

Embodiments of the present application will be described in detail below with reference to examples, but those skilled in the art will appreciate that the following examples are only for illustrating the present application and should not be construed as limitations to the scope of the present application. Experimental procedures without specified conditions in the examples are conducted according to conventional conditions or conditions recommended by the manufacturer. Reagents or instruments without specified manufacturers used herein are conventional products that are commercially available.

### Example 1: Antigen Protein Sequence Optimization and In Vitro Transcription (IVT)

### 1.1 Antigen protein sequence optimization

According to the sequence of the natural coding region of rabies virus G protein, the present application designed an SY mRNA sequence. In addition to the coding region, the features of the SY mRNA sequence also included an alpha-1-globin 5' UTR, gp130 and DH143 3' UTRs, and 100 polyAs. The SY-1, SY-2, and SY-3 mRNA sequences were all optimized in the G protein coding region. Compared to the original sequence of the rabies virus G protein, the GC contents of the optimized sequences were all increased, but remained consistent in the UTR region.

The specific experimental procedures are as follows: A plurality of optimized sequences were designed in the present disclosure and cloned into the vector pVAX.1 (GenScript Biotech Corporation). With the linearized vector (customized by GenScript Biotech Corporation) as a template, the mRNAs were synthesized *in vitro* and transfected into COS7 cells. At set time points, the expression levels of the G protein in cells were assayed by a flow cytometer to evaluate the effect of codon optimization on mRNA expression level and stability. The expression level results are as follows:

**Table 1. G protein expression level data for different codon optimization strategies**

| mRNA | 5'UTR | ORF | 3'UTR | polyA sequence and subsequent elements | Relative expression level |
|---|---|---|---|---|---|
| SY-0 1 SEQ ID NO.1 | pVAX.1+alp ha-1-globin 5'UTR | G protein nucleotide sequence optimization scheme 1 | gp130 and DH143 3' UTR | 100A | 30.39 |
| SY-02 SEQ ID NO.2 | pVAX.1+alp ha-1-globin 5'UTR | G protein nucleotide sequence optimization scheme 2 | gp130 and DH143 3' UTR | 100A | 26.74 |
| SY-03 SEQ ID NO.3 | pVAX.1+alp ha-1-globin 5'UTR | G protein nucleotide sequence optimization scheme 3 | gp130 and DH143 3' UTR | 100A | 28.06 |

| | | | | | |
|---|---|---|---|---|---|
| Note: The relative expression level means that: COS7 cells separately transfected with SY-01, SY-02, and SY-03 mRNAs were used as the experimental groups, and cells not transfected with mRNAs were used as the control group. The expression level of the G protein was measured *in vitro* according to the method in Example 3, and the mean fluorescence intensities (MFI) of the cells in the experimental groups and the cells in the control group were recorded, respectively. The relative expression level was calculated according to the following formula: | | | | | |

$Relative expression level = \frac{Experimental group MFI}{Control group MFI}$

According to the experimental results described above, SY-01 (SEQ ID NO. 1) with the highest relative expression level was selected as the preferred mRNA sequence for subsequent vaccine development and the sequence used in the following examples.

### 1.2 In vitro transcription (IVT) of G protein antigen

The procedures are as follows:
1. An IVT reaction system was prepared according to the instructions of an IVT kit (a kit from Vazyme, Catalog No. DD4201-P-01), that is, 10× Transcription Buffer, ATP, GTP, CTP, PseudoUTP (1-methyl-pseudouridine, Hongene Biotech, Catalog No. R5-064), a 5' cap analog (m7G(5')ppp(5')(2'OMeA)pG, Hongene Biotech, Catalog No. ON-134), water for injection, a plasmid template (a linearized plasmid with T7 promoter, the template being the DNA sequence corresponding to SEQ ID NO. 1, i.e., SEQ ID NO: 10), and Enzyme Mix were mixed.
2. The mixed reaction system was left to react at 37 °C for 40 min.
3. The reaction was terminated by adding DNase I at a corresponding ratio.
4. An equal volume of a LiCl solution at a certain concentration (7.5 M) was added for settling twice, and then the mixture was washed twice with 75% ethanol solution, dried in air at room temperature in a super clean bench, and dissolved in an appropriate amount of RNase-free H₂O.

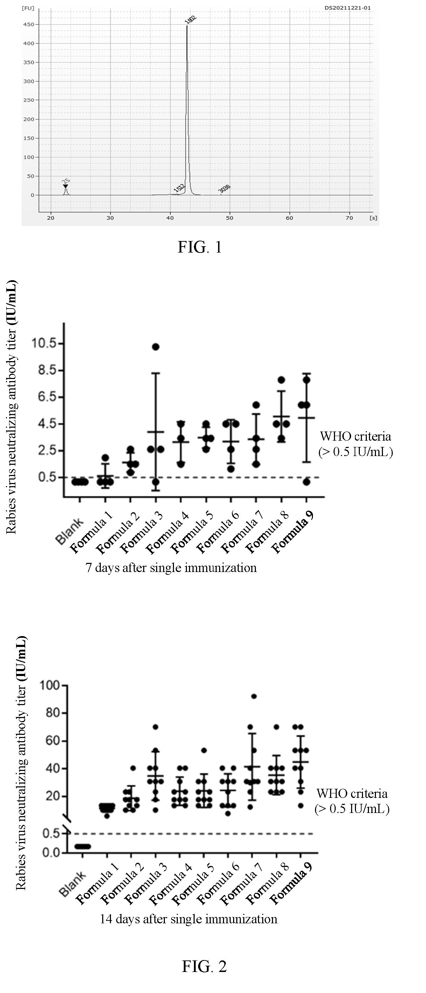

The purity of the resulting mRNA was analyzed by using a Bioanalyzer. The experimental results (see FIG. 1) show that after *in vitro* synthesis of the G protein mRNA, a high-purity mRNA was obtained by separation and purification.

### Example 2: Preferred Formulas and Preparation Methods for mRNA-LNP Formulations

### 2.1 Formula screening experiment

The formulation formula was optimized by comprehensively considering the following four aspects: lipid ratio, buffer system, N/P (nitrogen-to-phosphorus ratio), and whether lyophilization was performed. The specific formula selections are shown in Tables 2.1 and 2.2.

Normal mice were immunized with a single dose of 5 µg of mRNA (encapsulated in LNPs of different formulas 1-9), and then neutralizing antibodies in the serum of the mice were detected on days 7 and 14. Formula 9 was finally determined to be a preferred formula (see FIG. 2) according to the titer performance of neutralizing antibodies.

**Table 2.1. List of LNP formula components**

| No. | Ratio | Buffer system | N/P | Category | Treatment before administration |
|---|---|---|---|---|---|
| Formula 1 | C2 | PBS | 6 | After lyophilization | Add 400 µL of water for injection for reconstitution, and gently shake. |
| Formula 2 | C2 | PBS | 6 | Before | Be taken out at 20 °C, placed at room |
| | | | | lyophilization | temperature, thawed naturally, and gently shaken. |
| Formula 3 | A2 | PBS | 6 | Before lyophilization | |
| Formula 4 | C1 | PBS | 6.5 | Before lyophilization | |
| Formula 5 | A2 | TRIS | 6.5 | Before lyophilization | |
| Formula 6 | B4 | TRIS | 6.5 | Before lyophilization | |
| Formula 7 | B4 | TRIS | 6 | Before lyophilization | |
| Formula 8 | B6 | TRIS | 6.5 | Before lyophilization | |
| Formula 9 | A3 | TRIS | 6 | Before lyophilization | |

**Table 2.2. List of lipid ratios**

| | Ratio | MC3 | SM-102 | Cholesterol | PEG-DMG2000 | DSPC |
|---|---|---|---|---|---|---|
| | C2 | 0.5 | 0 | 0.4 | 0.03 | 0.07 |
| Molar ratio | A2 | 0 | 0.463 | 0.427 | 0.016 | 0.094 |
| | B4 | 0 | 0.463 | 0.413 | 0.03 | 0.094 |
| | B6 | 0 | 0.477 | 0.413 | 0.016 | 0.094 |
| | A3 | 0 | 0.5 | 0.385 | 0.015 | 0.1 |

### 2.2 Preparation method for mRNA-LNP formulations

Taking formula 9 described above as an example, the preparation procedures of a G protein antigen mRNA-LNP formulation are as follows:
1. Certain amounts of SM-102, DSPC, cholesterol, and DMG-PEG2000 lipid (i.e., at a molar mass ratio of 50%, 10%, 38.5%, and 1.5%, respectively) were each precisely weighed out and dissolved in an appropriate amount of absolute ethanol to prepare a lipid working solution for later use (the final concentration of the lipid working solution was 12 mg/mL);
2. a citric acid buffer (10 mM, pH 4.0) containing 130 mM sodium chloride, a Tris-NaOAc buffer (20 mM, 10.7 mM, pH 7.5), and a Tris-NaOAc buffer (20 mM, 10.7 mM, pH 7.5) containing 60% sucrose were each prepared;
3. an appropriate amount of an mRNA stock solution (the sequence is set forth in SEQ ID NO. 1) was diluted with the sodium chloride-citric acid buffer prepared above to adjust the final concentration of the mRNA working solution to 0.18 mg/mL;
4. the lipid working solution and the mRNA working solution were mixed in a volume ratio of 1:3 using a microfluidic instrument and a matched chip to prepare an mRNA-loaded LNP solution;
5. the Tris-NaOAc buffer was added in an amount of 9 times the volume of the prepared LNP solution for dilution, and the dilution was concentrated and purified by TFF (tangential flow ultrafiltration) to remove the ethanol solution from the system; and
6. the mRNA content in the LNP solution was determined by the ultraviolet spectrophotometry method, and an appropriate amount of the Tris-NaOAc buffer (20 mM, 10.7 mM, pH 7.5) containing 60% sucrose was added to adjust the final concentration of the mRNA in the finished LNP solution to 100 µg/mL, while the sucrose content in the external aqueous phase system was 8.7%.

The experimental results (see FIG. 3) show that the G protein mRNA and the lipid working solution were mixed by microfluidics to form the LNP complex with uniform particle size, mostly about 100 nm, which met the basic requirements for the next step.

### Example 3: In Vitro Expression Assay of G Protein

### 3.1 Western blotting assay:

The G protein mRNA was transfected into COS-7 cells (note: African green monkey kidney fibroblasts, purchased from Nanjing Cobioer Biosciences Co., Ltd.). 24 h after transfection, the cells were collected and subjected to the western blotting assay. Cells not transfected with the G protein mRNA were used as a negative control (Blank). The experimental results (see FIG. 4) show that the mRNA encoding the G protein obtained by the present application could be effectively and stably expressed in COS-7 cells at high levels.

### 3.2 Assay by flow cytometer:

After the G protein mRNA was transfected into 293T cells or COS-7 cells, the expression of the target antigen protein in the cells was assayed using an FACS flow cytometer. Cells not transfected with the G protein mRNA were used as a negative control (Blank). The specific method is described as follows (taking COS-7 cells as an example):
1. To detect the expression of the rabies virus G protein mRNA in human cells, COS-7 cells cultured for 24 h or more were digested and transferred into a 6-well plate, with the cell density controlled to be 250,000 cells/well.
2. After the six-well plate was incubated at 37 °C for 24 h, the cell state was observed using a microscope. When the cell confluence reached 80% or more, mRNA transfection could be performed.
3. The corresponding mRNA was transfected into COS-7 cells using a Lipofectamine 2000 kit (4 µg of mRNA was transfected into each well), and the specific procedures were referred to the kit instructions. The cells were further cultured at 37 °C for 24 h.
4. After 24 h of continuous mRNA expression, the expression level was close to the peak value; at this point, the cell supernatant was removed; and the cells were washed once with PBS, digested with 0.05% trypsin for 1 min, neutralized with a complete medium, and collected. The collected cells were centrifuged at 350 g for 5 min, and the supernatant was discarded. Then the cells were resuspended in 2 mL of PBS, collected, and centrifuged at 350 g for 5 min, and the supernatant was discarded. Finally, the cells were resuspended in 100 µL of PBS, with the cell number controlled to be 200,000 to 1,000,000.
5. The collected cells were fixed with 150 µL of Cyto-Fast^{™} Fix/Perm Buffer and incubated on ice for 20 min.
6. 1 mL of 1× Cyto-Fast^{™} Perm Wash solution prepared in advance was added to the fixed cells, and the mixture was centrifuged at 350 g for 5 min. The supernatant was discarded, and the cells were then resuspended in 1 mL of 1× Cyto-Fast^{™} Perm Wash solution, rinsed once again, and centrifuged to discard the supernatant.
7. The primary antibody was diluted to 1:100 with 100 µL of 1× Cyto-Fast^{™} Perm Wash solution; the dilution was added to the cells; and the cells were well mixed by shaking and incubated on ice for 30 min.
8. 1 mL of 1× Cyto-Fast^{™} Perm Wash solution was added to the cells incubated with the primary antibody, and the mixture was centrifuged at 350 g for 5 min. The supernatant was discarded, and the cells were then resuspended in 1 mL of 1× Cyto-Fast^{™} Perm Wash solution, rinsed once again, and centrifuged to discard the supernatant.
9. The secondary antibody was diluted to 1:1000 with 250 µL of 1× Cyto-Fast^{™} Perm Wash solution; the dilution was added to the cells; and the cells were well mixed by shaking and incubated on ice for 30 min.
10. 1 mL of 1× Cyto-Fast^{™} Perm Wash solution was added to the cells incubated with the secondary antibody, and the mixture was centrifuged at 350 g for 5 min. The supernatant was discarded, and the cells were then resuspended in 1 mL of 1× Cyto-Fast^{™} Perm Wash solution, rinsed once again, and centrifuged to discard the supernatant.
11. A 4% paraformaldehyde fixative solution was diluted to a 1% paraformaldehyde fixative solution with PBS. Finally, the cells were resuspended in 200 µL of the 1% paraformaldehyde fixative solution, and the expression of the rabies glycoprotein in the cells was assayed on a flow cytometer.
12. Flow cytometry: Cells not transfected with the mRNA were set as a negative control group; the samples were sequentially assayed; and FITC fluorescence intensity signals were read using the histogram. The mean fluorescence intensity (MFI) of the cells was recorded, and 10,000 signals were read for each sample.

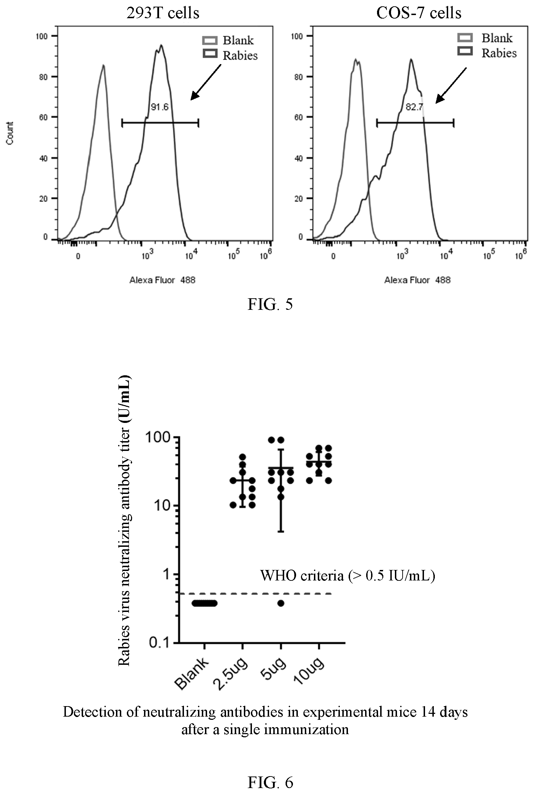

The experimental results (see FIG. 5) show that the mRNA encoding the G protein obtained by the present disclosure could be effectively and stably expressed in COS-7 cells and 293T cells.

### Example 4: Detection of Neutralizing Antibodies in Mice

Healthy BALB/c mice (purchased from Shanghai Model Organisms Center, Inc.) aged 6-8 weeks in a good growth state were selected and randomly divided into groups of 10 (half male and half female) after body weight measurement. The administration was performed by intramuscular injection (the mRNA delivered by LNP prepared in Example 2.2). The administration was performed on day 0, and the administration doses were 2.5 µg, 5 µg, and 10 µg.

Different doses of the G protein mRNA (2.5 µg, 5 µg, and 10 µg) were used to immunize the mice, and after 14 days, neutralizing antibodies in the serum of the mice were detected. The experimental results (see FIG. 6) show that high concentrations of neutralizing antibodies were detected in the serum of the mice even in the low-dose group. Moreover, neutralizing antibodies in these groups were all much higher than the criteria of 0.5 IU/mL specified by WHO.

### Example 5: Detection of Neutralizing Antibodies in Mice Immunized by Different Immunization Procedures

Healthy BALB/c mice (purchased from Shanghai Model Organisms Center, Inc.) aged 6-8 weeks in good growth were selected and randomly divided into groups of 10 (half male and half female) after body weight measurement. The administration was performed by intramuscular injection (the mRNA delivered by LNP prepared in Example 2.2). The administration was performed on day 0, days 0 and 3, and days 0 and 7. The administration doses were 0.125 µg, 0.5 µg, 1 µg, and 2 µg (by injection on day 0); 0.0625 µg, 0.25 µg, and 1 µg (by injection on days 0 and 3); and 1 µg (by injection on days 0 and 7). Normal mice were immunized with different doses (0.125 µg, 0.5 µg, 1 µg, and 2 µg, as well as 0.0625 µg, 0.25 µg, and 1 µg) and by different immunization modes (0D injection, 0/3D injection, and 0/7D injection). After 5 days and 14 days, neutralizing antibodies in the serum of the mice were detected.

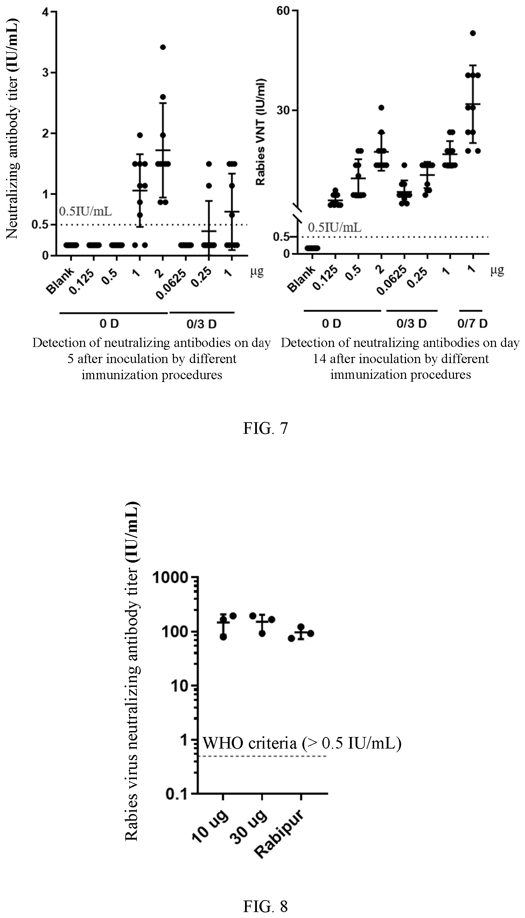

The experimental results (see FIG. 7) show that under the condition of a single injection of 1 µg of mRNA, neutralizing antibodies higher than the WHO criteria (0.5 IU/mL) could be detected in the mice after 5 days. Under the condition of two injections of 0.0625 µg of mRNA on days 0/3, neutralizing antibodies higher than the WHO criteria (0.5 IU/mL) were also detected in the mice on day 14. It can be seen that the mRNA vaccines of the present application can also produce high levels of neutralizing antibodies at an extremely low injection dose (0.0625 µg).

### Example 6: NIH Potency Test

The NIH potency of the finished mRNA vaccine formulation (the test vaccine) prepared in Example 2.2, the positive reference mRNA vaccine (prepared with reference to the preparation method for the rabies virus nucleic acid vaccine in Example 1 of the patent CN110714015A), and the reference vaccine standard (the standard vaccine of the National Institutes for Food and Drug Control is an inactivated vaccine, with a calibrated potency of 11.4 IU/mL) was determined according to the standard NIH potency determination procedures specified by WHO. The specific procedures are as follows:
1. One vial of lyophilized reference vaccine was taken, 1.0 mL of sterile water for injection was added, and the mixture was used after being completely dissolved and well mixed; the labeled potency after reconstitution was 11.4 IU/mL; and the reconstituted reference vaccine was serially 5-fold diluted with PBS (pH 7.4), and samples at 4 dilutions of 1:25, 1:125, 1:625, and 1:3125 were taken and stored in an ice bath for later use.
2. Samples of the test vaccine and the positive reference vaccine were serially 5-fold diluted, and samples at 5 dilutions including the finished vaccine product, 1:5, 1:25, 1:125, and 1:625 were taken and stored in an ice bath for later use.
3. At the first immunization, the diluted reference vaccine and test vaccine at each dilution were taken for inoculation from the highest dilution to the lowest dilution, and 16 female mice aged 3-4 weeks and weighing 11-16 g were intraperitoneally injected with the vaccine at each dilution at 0.5 mL/mouse. One week after the first immunization, the reference vaccine and the test vaccine at each dilution were taken, and the mice after the first immunization were subjected to the second immunization according to the aforementioned method and dose.
4. 14 days after the first immunization, all immunized mice were inoculated intracerebrally with 50 LD50/0.03 mL rabies virus CVS-24 strain virus solution at 0.03 mL/mouse. All mice were observed for 14 consecutive days after challenge. The death of mice within the first 4 days (including day 4) was recorded as non-specific death, the number of mice dying of rabies in each group every day was recorded from day 5, and the mice showing typical rabies symptoms such as paralysis or spasm on day 14 were also counted as dead. The number of mice that died and survived within 5-14 days was statistically analyzed.
5. The ED50 values of the test vaccine and the reference vaccine were calculated using the Reed-Muench method, and the relative potency (RP, i.e., NIH potency) of the test vaccine was calculated according to the following formula. $RP = \frac{Reciprocal of ED 50 of test vaccine}{Reciprocal of ED 50 of reference vaccine} \times Reference vaccine potency \left(IU/mL\right)$

The specific results are as follows:

**Table 3. Results of NIH potency test**

| **Group** | **Dilution ratio** | **Survival rate** | | **Mortality** | **NIH Potency** |
|---|---|---|---|---|---|
| | 1:25 | 14/15 | 93.33% | 6.67% | |
| | 1:125 | 12/16 | 75% | 25% | |
| Test mRNA vaccine | 1:625 | 10/16 | 62.5% | 37.5% | 25.49 IU/mL |
| | 1:3125 | 3/16 | 18.75% | 81.25% | |
| | 1:15625 | 0/16 | 0% | 100% | |
| | 1:25 | 15/16 | 93.75% | 6.25% | |
| | 1:125 | 10/16 | 62.5% | 37.5% | |
| Positive reference mRNA vaccine | 1:625 | 6/16 | 37.5% | 62.5% | 9.71 IU/mL |
| | 1:3125 | 1/15 | 6.67% | 93.33% | |
| | 1:15625 | 0/16 | 0% | 100% | |
| | 1:25 | 15/16 | 93.75% | 6.25% | |
| Reference vaccine (standard) | 1:125 | 10/16 | 62.5% | 37.5% | 11.4 IU/ml |
| | 1:625 | 6/16 | 37.5% | 62.5% | |
| | 1:3125 | 3/16 | 5% | 95% | |

The results described above show that the potency of the rabies mRNA vaccine obtained by the present application was measured to be 25.49 IU/mL, which was much higher than that of the positive reference mRNA vaccine (9.71 IU/mL) and the reference vaccine (11.4 IU/mL). In addition, the mortality of mice immunized with the rabies mRNA vaccine of the present application was much lower than that of mice immunized with the positive reference vaccine at the same dose.

### Example 7: LNP Formulation Formula Stability Experiment

The finished rabies mRNA vaccine formulation (prepared by the method in Example 2.2) was stored under the conditions of -20 °C and 5 °C, and the stability of the vaccine was investigated according to different physicochemical indexes. After storage for six months under the two conditions, the physicochemical properties of the finished mRNA vaccine formulation were not significantly changed, the RNA concentration was not significantly changed, and the encapsulation efficiency was kept 75% or above. The above results show that the vaccine formulation had long-term stability. See Table 4 for details:

**Table 4. LNP vaccine stability experimental results**

| **Duration of investigation** | **0 M(Month)** | **1 M** | | **5 M** | | **6 M** | |
|---|---|---|---|---|---|---|---|
| **Type of stability investigation** | Complete inspection | Long-term experiment (-20 °C) | Accelerated experiment (5 °C) | Long-term experiment | Accelerated experiment | Long-term experiment | Accelerated experiment |
| **Appearance** | Milky white clear liquid | Milky white clear liquid | Milky white clear liquid | Milky white clear liquid | Milky white clear liquid | Milky white clear liquid | Milky white clear liquid |
| **Osmotic pressure** | 365 | 345 | 343 | 345 | 351 | 349 | 350 |
| **PH** | 7.5 | 7.5 | 7.5 | 7.3 | 7.4 | 7.3 | 7.3 |
| **Insoluble particle** | ≥10um: 110 ≥25um: 4 | ≥10um: 133 ≥25um: 5 | ≥10um: 312 ≥25um: 3 | ≥10um: 239 ≥25um: 4 | ≥10um: 367 ≥25um: 13 | ≥10um: 394 ≥25um: 7 | ≥10um: 407 ≥25um: 19 |
| **mRNA concentration (µg/mL)** | 69 | 68 | 67 | 68 | 66 | 71.9 | 72.8 |
| **Encapsulation efficiency (%)** | 89 | 80 | 79 | 84 | 84 | 82 | 81 |
| **Particle size (nm)** | 110.9 | 126.5 | 129.5 | 133 | 126 | 132 | 124 |
| **Potential (mv)** | -7.27 | -2.87 | -7.85 | -3.8 | 0 | -2.5 | -2.4 |
| **Purity (%)** | 96 | 92 | 93 | 92 | 86 | 91 | 81.1 |

### Example 8: Experiment on Neutralizing Antibodies in Cynomolgus Monkeys

Normal cynomolgus monkeys (3 monkeys per dose group) were immunized twice with different doses (10 µg and 30 µg) of LNP formulations of the mRNA vaccine (prepared by the method in Example 2.2) on days 0/7, and neutralizing antibodies in the serum of the cynomolgus monkeys were detected on day 14. The experimental results (see FIG. 8) show that compared with the marketed Rabipur, the rabies mRNA vaccine of the present disclosure produced higher titers of neutralizing antibodies with fewer immunizations.

### Example 9: Post-Exposure Immunization Experiment

Mice were immunized twice with 3 µg of the LNP formulation of the mRNA vaccine (prepared by the method in Example 2.2) on days 0 and 3, respectively, after challenge, and the survival rates of the mice in each group on days 7, 14, and 21 were investigated. The protective efficacy of the mRNA vaccine of the present application was significantly higher than that of the standard positive reference mRNA vaccine (prepared with reference to the preparation method for the rabies virus nucleic acid vaccine in Example 1 of the patent CN110714015A). The specific results are shown in Table 5.1:

**Table 5.1. Results of post-exposure immunization experiment**

| **Immunization procedure** | **Drug** | **Dose/mouse** | **7-day survival rate** | **14-day survival rate** | **21-day survival rate** |
|---|---|---|---|---|---|
| Inoculated with 2 doses of vehicle on days 0 and 7 after challenge | Vehicle group | | 10/10 | 1/10 | 0/10 |
| Inoculated with 4 doses on days 0, 3, 7, and 14 after challenge | Inactivated Vaccine, rabies standard from the National Institutes for Food and Drug Control | 0.57 IU | 10/10 | 4/10 | 4/10 |
| Inoculated with 2 doses on days 0 and 3 after challenge | Test mRNA vaccine | 3 µg | 10/10 | 7/10 | 7/10 |
| Inoculated with 2 doses on days 0 and 3 after challenge | Positive reference mRNA vaccine | 3 µg | 10/10 | 3/10 | 2/10 |

In addition, the present disclosure also performed related tests on the mRNA vaccine of the present application and the inactivated vaccine Rabipur^{®}. Female BALB/c mice, 10 mice per group, were selected for post-exposure immunization, and the survival of the animals was observed until D28 after inoculating the mRNA vaccine and Rabipur^{®} on the day of challenge (D0). The specific results are shown in Table 5.2.

**Table 5.2. Survival rate results of post-exposure immunization**

| **Vaccine** | Immunization dose | Immunization strategy | Survival rate% |
|---|---|---|---|
| Rabipur^{®} | 1/10 dose | 0, 3, 7, 14 | 60 |
| | 1/30 dose | | 0 |
| mRNA vaccine | 1/10 dose | 0, 7 | 100 |
| | 1/30 dose | | 80 |

| | | | |
|---|---|---|---|
| Note: 1. The specification of the Rabipur inactivated vaccine for human use was 300 µL, and the dose for injection into mice was 1/10 and 1/30 of the dose for human use; 2. The provisional specification of the mRNA vaccine for human use of the present application was 30 µg, and the dose for injection into mice was 1/10 and 1/30 of the dose for human use. | | | |

It can be seen that the survival rate of the mice injected with the mRNA vaccine of the present application was significantly higher than that of the Rabipur group, demonstrating that the immune effect of the vaccine of the present application is significantly superior to that of Rabipur.

### Example 10: Hepatotoxicity Experiment on Rabies mRNA Vaccine

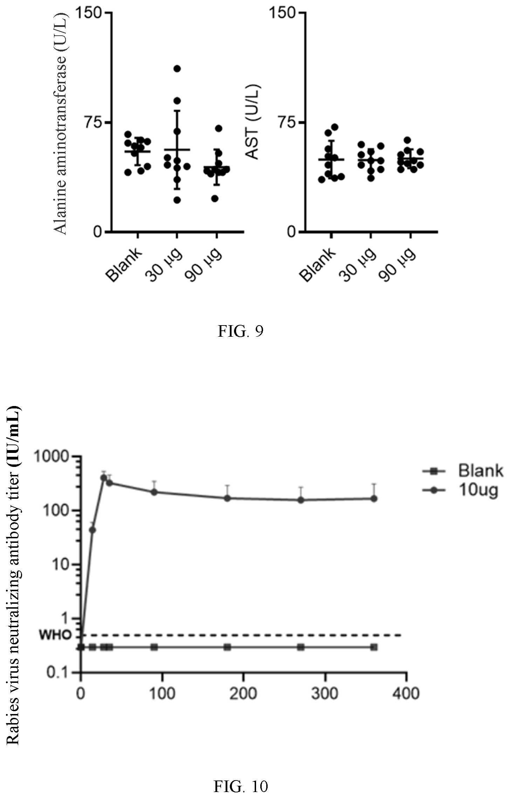

The cynomolgus monkeys were immunized 3 times on days 0, 7, and 14 with low-dose (30 µg) and high-dose (90 µg) LNP formulations of the mRNA vaccine (prepared by the method in Example 2.2). On day 17, the alanine aminotransferase activity and the aspartate aminotransferase activity in the liver of the cynomolgus monkeys in each group were investigated. The results (see FIG. 9) show that regardless of the low-dose group or the high-dose group, the rabies mRNA vaccine prepared in the present disclosure did not exhibit significant hepatotoxicity, and had very good safety.

### Example 11: Experiment on Long-Term Effectiveness of Rabies mRNA Vaccine

Balb/C mice aged 6-8 weeks were selected, with 10 mice in each group (5 male and 5 female), and administered by intramuscular injection. 10 µg of the vaccine was injected on days 0 and 21. Then the serum of the mice was collected on days 0, 14, 28, 35, 90, 180, 270, and 360, and the concentration of neutralizing antibodies in the serum of the mice was determined. The results (see FIG. 10, the abscissa represents days) show that after the injection of 10 µg of the mRNA vaccine LNP formulation (prepared by the method in Example 2.2) on days 0 and 21, high concentrations of neutralizing antibodies could be produced in the mice for a long time (at least one year), and the concentrations were much higher than the criteria of 0.5 IU/mL specified by WHO. Thus, the vaccine had a long-acting protection effect on the rabies virus.

### Example 12: Experiment on Neutralizing Activity of Rabies mRNA Vaccine Against Chinese Mainstream Strains

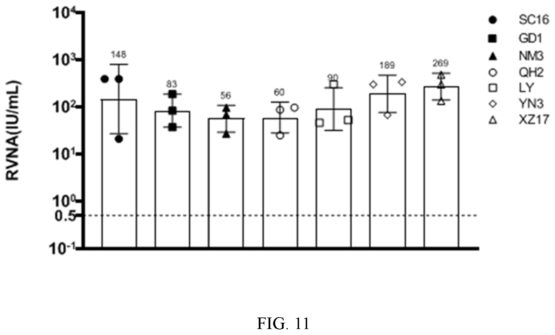

The mRNA vaccine LNP formulation was intramuscularly injected into cynomolgus monkeys, and the first immunization of the animals was recorded as D0. The animals were immunized twice at an interval of 7 days (D0 and D7). The monkey serum was collected 14 days after the 2 immunizations (D21), and the *in vitro* neutralizing activity of the serum against 7 representative strains of China populations, such as SC16, GD1, NM3, QH2, LY, YN3, and XZ17, was detected (FIG. 11). The results of molecular epidemiological studies show that rabies viruses prevalent in China can be divided into the seven populations described above (SC16, GD1, NM3, QH2, LY, YN3, and XZ17). The experimental results show that the neutralizing antibody positive conversion rates (GMT > 0.5 IU/mL) of all strains for all animals were 100%, suggesting that the mRNA vaccine of the present application has broad-spectrum neutralizing activity against the rabies virus.

The experimental results described above show that the mRNA vaccine obtained by the present disclosure is able to produce a high level of neutralizing antibodies and effectively induce B cell immunity and T cell immunity, and the comprehensive immune effect thereof is far superior to that of the positive reference control. In addition, the storage conditions for the vaccine are moderate, and the vaccine can be stably stored for a long time at -20 °C, has relatively low requirements for equipment due to relatively convenient transportation, and is more suitable for regions such as economically undeveloped or under-developed regions where vaccination is urgently needed. In addition, the rabies mRNA vaccine obtained by the present disclosure has broad-spectrum neutralizing activity against representative strains of seven populations of rabies viruses prevalent in China, and provides a relatively good protection effect for challenge mice in pre-exposure and post-exposure immunization experiments.

## Claims

1. An mRNA encoding a rabies virus antigen, wherein the rabies virus antigen comprises an amino acid sequence having at least 80%, at least 85%, at least 90%, at least 95%, at least 98%, or 100% identity to the amino acid sequence of SEQ ID NO. 4.

2. The mRNA according to claim 1, wherein the mRNA comprises a 5' UTR, a 3' UTR, and 3'-polyadenylic acid.

3. The mRNA according to claim 2, wherein the 3'-polyadenylic acid comprises the nucleotide sequence of SEQ ID NO. 7.

4. The mRNA according to any one of claims 2-3, wherein the 5' UTR comprises the nucleotide sequence of SEQ ID NO. 6, and/or the 3' UTR comprises the nucleotide sequence of SEQ ID NO. 8 and/or SEQ ID NO. 9.

5. The mRNA according to any one of claims 2-4, comprising a nucleotide sequence having at least 80%, at least 85%, at least 90%, at least 95%, at least 98%, or 100% identity to the nucleotide sequence of any one of SEQ ID NOs. 1-3.

6. The mRNA according to any one of claims 1-5, wherein the mRNA further comprises a 5' guanosine cap selected from: m7Gppp(2'OMeA)pG, m7GpppApA, m7GpppApC, m7GpppApG, m7GpppApU, m7GpppCpA, m7GpppCpC, m7GpppCpG, m7GpppCpU, m7GpppGpA, m7GpppGpC, m7GpppGpG, m7GpppGpU, m7GpppUpA, m7GpppUpC, m7GpppUpG, m7GpppUpU, m7Gpppm6ApG, m7G3'OmepppApA, m7G3'OmepppApC, m7G3'OmepppApU, m7G3'OmepppApG, m7G3'OmepppCpA, m7G3'OmepppCpC, m7G3'OmepppCpG, m7G3'OmepppCpU, m7G3'OmepppUpA, m7G3'OmepppUpC, m7G3'OmepppUpG, m7G3'OmepppUpU, m7G3'OmepppA2'OmepG, m7G3'OmepppA2'OmepC, m7G3'OmepppA2'OmepU, m7G3'OmepppA2'OmepA, m7G3'OmepppC2'OmepA, m7G3'OmepppC2'OmepU, m7G3'OmepppC2'OmepG, m7G3'OmepppC2'OmepC, m7G3'OmepppG2'OmepA, m7G3'OmepppG2'OmepU, m7G3'OmepppG2'OmepG, m7G3'OmepppG2'OmepC, m7G3'OmepppU2'OmepA, m7G3'OmepppU2'OmepU, m7G3'OmepppU2'OmepG, and m7G3'OmepppU2'OmepC.

7. The mRNA according to any one of claims 1-6, wherein the rabies virus antigen encoded by the mRNA is a G protein.

8. A composition comprising a lipid nanoparticle and an mRNA, wherein the mRNA is the mRNA according to any one of claims 1-7.

9. The composition according to claim 8, comprising an ionizable cationic lipid, a structural lipid, a helper lipid, and a surfactant.

10. The composition according to any one of claims 8-9, wherein the lipid nanoparticle comprises, by molar percentage (mol%), 20-60 mol% ionizable cationic lipid, 25-55 mol% structural lipid, 5-25 mol% helper lipid, and 0.5-15 mol% surfactant.

11. The composition according to any one of claims 8-10, wherein the structural lipid is selected from cholesterol and cholesterol derivatives, preferably cholesterol.

12. The composition according to any one of claims 8-11, wherein the cationic lipid is selected from: SM-102, ALC-0315, ALC-0519, Dlin-MC3-DMA, DODMA, DLin-KC2-DMA, and DlinDMA, preferably SM-102.

13. The composition according to any one of claims 8-12, wherein the helper lipid is selected from DSPC, DOPE, DOPC, DOPG, and DOPS, preferably DSPC.

14. The composition according to any one of claims 8-13, wherein the surfactant is selected from PEG2000-DMG, PEG-DSPE, DTDA-PEG2000, and TPGS, preferably PEG2000-DMG.

15. The composition according to any one of claims 8-14, wherein the lipid nanoparticle comprises, by molar percentage (mol%), 50 mol% SM-102, 10 mol% DSPC, 38.5 mol% cholesterol, and 1.5 mol% PEG2000-DMG.

16. The mRNA according to any one of claims 1-7 or the composition according to any one of claims 8-15, wherein the mRNA comprises a chemically modified base or analog thereof, and the chemically modified base or analog thereof is selected from 5-methoxymethyl uridine, 5-methylthio uridine, 1-methoxymethyl pseudouridine, 5-methyl cytidine, 5-methoxy cytidine, 1-methyl-pseudouridine (N1-methyl-pseudoUTP), and pseudouridine, preferably 1-methyl-pseudouridine.

17. Use of the mRNA according to any one of claims 1-7 or the composition according to any one of claims 8-16 in the preparation of a rabies virus nucleic acid vaccine.

18. The use according to claim 17, wherein the vaccine comprises a combination vaccine and a multivalent vaccine.

19. The use according to any one of claims 17-18, wherein the vaccine is an anti-rabies virus mRNA vaccine.

20. A method for pre-exposure or post-exposure immunization, comprising administering to a subject the composition according to any one of claims 8-16, wherein said administration is effective to induce an immune response against a rabies virus antigen in the subject.

21. A pharmaceutical composition, comprising an mRNA which comprises the sequence set forth in SEQ ID NO. 1, a lipid nanoparticle composition, 20 mmol/L tromethamine, 10.7 mmol/L sodium acetate, and 8.7% sucrose, with a pH of 7.0-8.0, wherein the lipid nanoparticle comprises 50 mol% SM-102, 10 mol% DSPC, 38.5 mol% cholesterol, and 1.5 mol% PEG2000-DMG, and with a nitrogen-to-phosphorus ratio (N:P) of 6.

22. A preparation method for a nucleic acid vaccine, comprising the following steps:
(1) dissolving a protonatable cationic lipid, a structural lipid, a helper lipid, and a surfactant in an organic solution in a specific ratio to obtain an organic phase;
(2) dissolving an mRNA having the sequence set forth in SEQ ID NO. 1 in a citrate buffer or a sodium acetate solution to obtain an aqueous phase; and
(3) uniformly mixing the organic phase in step (1) and the aqueous phase in step (2) to produce a mixed solution, so as to obtain a rabies virus vaccine.

23. The preparation method according to claim 22, wherein the organic solution comprises absolute ethanol.

24. The preparation method according to any one of claims 22-23, wherein the total concentration of the protonatable cationic lipid, the structural lipid, the helper lipid, and the surfactant in the organic phase is 10 to 15 mg/mL.

25. The preparation method according to any one of claims 22-24, wherein the concentration of the mRNA is 0.01 to 1 mg/mL, preferably 0.1 to 0.2 mg/mL.

26. The preparation method according to any one of claims 22-25, wherein the organic phase and the aqueous phase are in a volume ratio of 1:(2 to 4).

27. The preparation method according to any one of claims 22-26, wherein uniformly mixing is performed by adopting a microfluidic device, with a flow rate controlled to be ≥ 12 mL/min.

28. A nucleic acid molecule set forth in SEQ ID NO. 10.

29. A biomaterial expressing the nucleic acid molecule according to claim 28, wherein preferably, the biomaterial comprises: an expression cassette, recombinant vector, recombinant plasmid, or transgenic cell line, each comprising the nucleic acid molecule; the recombinant vector is preferably a pVAX.1 vector or a pcDNA3.1 vector.

30. The mRNA according to any one of claims 1-7, the composition according to any one of claims 8-16, the use according to any one of claims 17-19, and the method according to claim 20, wherein a strain of the rabies virus comprises, but is not limited to: a Pasteur strain (PAS), a Flury strain, a SAD strain, an aG strain, a CTN strain, SC16 (CSC1016D), GD1 (GDZAQ45), NM3 (CNM1103C), QH2 (CQH1202D), LY (CNX8601), YN3 (CYN1009D), XZ17 (CXZ1704H), and derivative strains thereof, preferably a CTN strain, and most preferably a CTN-1 strain.
